# EUROPEAN PATENT SPECIFICATION

(11) **EP 3 838 279 B1**
(45) Date of publication and mention of the grant of the patent: **05.03.2025**
(21) Application number: 19849254.8
(22) Date of filing: 14.08.2019
(51) Int. Cl.: A61K 35/35, A61K 35/545, A61P 3/00, A61P 3/04, A61P 3/10, A61P 5/50, A61P 43/00, C12N 5/074

(54) **SUPERNATANT OF BROWN ADIPOCYTES, METHOD FOR PREPARING SAME AND UTILIZATION THEREOF**
ÜBERSTAND VON BRAUNEN ADIPOZYTEN, VERFAHREN ZU SEINER HERSTELLUNG UND SEINE VERWENDUNG
SURNAGEANT D'ADIPOCYTES BRUNS, SA MÉTHODE DE PRÉPARATION, ET SON UTILISATION

(30) Priority: 14.08.2018 JP 2018152727; 18.02.2019 JP 2019026463
(43) Date of publication of application: 23.06.2021
(73) Proprietor: National Center for Global Health and Medicine, Tokyo 162-8655 (JP); ID Pharma Co., Ltd., Tokyo 102-0071 (JP)
(72) Inventor: SAEKI, Kumiko, Tokyo 162-8655 (JP); KOBAYASHI, Norihiko, Tokyo 162-8655 (JP); OKA, Masako, Tokyo 162-8655 (JP); MATSUMURA, Kazunori, Tokyo 162-8655 (JP); NISHIO, Miwako, Tokyo 162-8655 (JP); SHU, Tsugumine, Tokyo 102-0071 (JP); MORI, Toyotaka, Tokyo 102-0071 (JP)
(74) Representative: Schiweck Weinzierl Koch Patentanwälte Partnerschaft mbB
(86) International application number: PCT/JP2019/031897
(87) International publication number: WO 2020/036184

(56) References cited:
- WO-A1-2012/147853
- WO-A1-2013/003595
- JP-A- 2018 108 080
- YONG CHEN ET AL: "Exosomal microRNA miR-92a concentration in serum reflects human brown fat activity", NATURE COMMUNICATIONS, vol. 7, 27 April 2016 (2016-04-27), pages 11420, XP055296084, DOI: 10.1038/ncomms11420
- NAKAHARA M ET AL: "Human Embryonic Stem Cells with Maintenance under a Feeder-Free and Recombinant Cytokine-Free Condition", CLONING AND STEM CELLS, MARY ANN LIEBERT, LARCHMONT, US, vol. 11, no. 1, 1 March 2009 (2009-03-01), pages 5 - 18, XP002730678, ISSN: 1536-2302, [retrieved on 20081217], DOI: 10.1089/CLO.2008.0043
- STANFORD KRISTIN I. ET AL: "Brown adipose tissue regulates glucose homeostasis and insulin sensitivity", THE JOURNAL OF CLINICAL INVESTIGATION, vol. 123, no. 1, 2 January 2013 (2013-01-02), GB, pages 215 - 223, XP055901189, ISSN: 0021-9738, Retrieved from the Internet <URL:https://dm5migu4zj3pb.cloudfront.net/manuscripts/62000/62308/cache/62308.2-20201218131621-covered-e0fd13ba177f913fd3156f593ead4cfd.pdf> DOI: 10.1172/JCI62308
- NISOLI, E. ET AL.: "Expression of Nerve Growth Factor in Brown Adipose Tissue: Implications for Thermogenesis and Obesity", ENDOCRINOLOGY, vol. 137, no. 2, February 1996 (1996-02-01), pages 495 - 503, XP055686688
- NISHIO, M. ET AL.: "Production of Functional Classical Brown Adipocytes from Human Pluripotent Stem Cells using Specific Hemopoietin Cocktail without Gene Transfer", CELL METABOLISM, vol. 16, 2012, pages 394 - 406, XP055142155, DOI: 10.1016/j.cmet.2012.08.001
- SAEKI,KUMIKO: "Production of brown adipocytes from human pluripotent stem cells", JOURNAL OF CLINICAL AND EXPERIMENTAL MEDICINE, vol. 250, no. 9, 1 September 2014 (2014-09-01), pages 816 - 821, XP009526444, ISSN: 0039-2359
- SAEKI,KUMIKO: "Function of brown adipocytes established from human ES/iPS cells", THE LIPID, vol. 25, no. 1, 2014, pages 74 - 80, XP009526443, ISSN: 0915-6607

## Description

### [Technical Field]

The present invention relates to a method of preparing a supernatant of brown adipocytes having actions such as promotion of insulin secretion, enhancement of glucose uptake by pancreatic beta cells, skeletal muscle cells, and myocardial cells, improvement of mitochondrial function, and/or promotion of healing of skin damage. The present invention also relates a composition comprising the supernatant of brown adipocytes, an internal medical treatment aimed at correcting metabolic disorders using the composition, and such.

### [Background Art]

The population of obese and overweight people is increasing worldwide. According to the Global Burden of Diseases, Injuries, and Risk Factors Study of 2017, one in three people in the world is obese or overweight (Non-Patent Literature (NPL) 1). Obesity and overweightness increase the risk of developing life-threatening group of diseases such as type 2 diabetes, ischemic heart disease, cerebrovascular disorders, and cancer. Furthermore, those who have experienced obesity or overweightness in the past have a higher risk of death than those who maintain normal body weight (NPL 2). It has also been reported that the higher the blood glucose level of a pregnant woman, infinitely larger the burden on the offspring (NPL 3), and moreover, that the child whose mother was obese or overweight in the early stages of pregnancy has a higher incidence of cerebral palsy (NPL 4), and children of obese and overweight pregnant women are prone to malformations (NPL 5). In this way, obesity and overweightness have a serious effect on the health of, obviously the individual, and also the next generation.

With the increase in the number of individuals affected with obesity/overweightness, the biggest threat is the rise in the number of people with type 2 diabetes. The prevalence of diabetes is estimated to be 9.5 million in Japan and 420 million worldwide. In a 2013 survey in China, the prevalence of diabetes in adults was estimated to be 10.9%, the prevalence of prediabetes was estimated to be 35.7% (NPL 6), and the risk of death for diabetic patients was twice as high as that for non-diabetic individuals (NPL 7). Furthermore, in a survey conducted in the United States from 1983 to 2011, the number of people affected with ischemic heart disease has decreased by about 20%, while the number of people affected with diabetes has increased (NPL 8). Further, in Mexico, about 1 in 4 people is diagnosed with diabetes (NPL 9).

Among diabetes, type 2 diabetes also has the issue of having a high incidence of complications. For example, a nearly eight-year follow-up of 2018 people diagnosed with diabetes by age 20 found that the prevalence of complications was 32% in type 1 diabetics and 72% in type 2 diabetics (NPL 10).

Thus, the increase in the number of individuals affected with obesity/overweightness and the accompanying increase in the prevalence of type 2 diabetes are urgent issues that require global countermeasures.

However, it is not easy for individuals affected with obesity/overweightness to lose weight and maintain their lost weight. Furthermore, a 1988-2014 survey in the United States reported a decrease in the proportion of "obese/overweight adults trying to lose weight" (NPL 11).

Currently, it is hard to say that the treatment of type 2 diabetes is sufficiently effective, and patient satisfaction with the treatment is also low. For example, in a February 2017 report in the United States, the percentage of diabetic patients who had 6% or lower glycated hemoglobin (HbA1c) was 29% in the patient group who had undergone surgery for obesity (gastric bypass group) at the 5-years time point, but was a mere 5% in the group treated with internal medications (NPL 12). Thus, obesity surgery outperforms treatment with internal medications in glycemic control in diabetic patients. However, obesity surgery is an invasive procedure, and the development of an excellent therapeutic agent for type 2 diabetes that surpasses the current therapeutic agents is anticipated.

Furthermore, a meta-analysis of prospective observational studies reported in 2016 showed that the risk of cardiovascular disease increased from HbA1c of 5.7% (NPL 13). This means that from the standpoint of completely preventing the onset of complications in type 2 diabetes, current internal medications are only taking inadequate measures in 95% of cases.

The way it is now is that a significant amount of research grants and medical expenses are being invested in early diagnosis and treatment of complications. However, the first priority should be to develop an excellent therapeutic agent that can control glycemic control to an HbA1c of 5.7% or less.

In fact, on January 30, 2017, a pharmaceutical company with the world's No. 1 sales record for diabetes drugs announced that it would partner with Oxford University in the UK to aim at discovering a new treatment method for type 2 diabetes. The company has also announced that it will set up a new research center on the university premises and will invest 115 million pounds over the next decade. This is one lurid illustration of the inadequacy of current internal medical treatment for type 2 diabetes.

As mentioned above, the development of a new therapeutic drug that enables strict blood glucose control (HbA1c ≤ 5.7%) in type 2 diabetes is an extremely important and urgent drug discovery enterprise. However, no matter how many times drug discovery research based on non-novel strategies based on the conventional understanding of the pathophysiology of type 2 diabetes is repeated, a new drug that achieves the above-mentioned goal will not be developed. In order to develop an excellent therapeutic drug with both high safety and efficacy, it is necessary to fundamentally review the understanding of the pathophysiology of type 2 diabetes.

From this viewpoint, "Brown Adipose Tissue (BAT)" deserves attention.

Brown adipocytes that constitute BAT rapidly decompose neutral fat stored in intracellular multilocular lipid droplets when the demand for body heat production increases, such as for maintaining body temperature in a cold environment. Using the fatty acids produced, uncoupled respiration in mitochondria releases heat energy, and thus, brown adipocytes contribute to the maintenance of body heat. BAT has been studied as adipose tissue that contributes to rapid body heat production after hibernation in small hibernating animals, but it was reported in 2009 that BAT also exists in human adults (NPL 14-17).

Studies using genetically modified mice have demonstrated that BAT has the actions of "preventing obesity," "improving glucose metabolism," "improving lipid metabolism," and "enhancing leptin sensitivity." Moreover, in humans also, epidemiological studies using data from cancer screenings and health checks, and healthy volunteer studies suggest that BAT contributes to "prevention of middle-aged overweightness" and "improvement of glucose metabolism".

Initially, the metabolism-improving action of BAT was thought to be a secondary effect of weight loss as a result of calorie consumption by fat burning. However, brown adipocyte-deficient mice exhibited severe obesity and diabetes, whereas mice lacking only the heat-producing ability of brown adipocytes did not exhibit obesity or glucose metabolism disorders. This suggested that the improvement of glucose metabolism by brown adipocytes is not a secondary effect mediated by heat production, but an "action mediated by a soluble factor (hormone)". The "glucose metabolism-improving hormone produced by brown adipocytes" was collectively called BATokine, and research aimed at its identification was propelled worldwide. However, to date, a "true BATokine", which is specifically produced by brown adipocytes or whose main source of origin is brown adipocytes, has not been identified. For example, cytokines such as interleukin 6 (IL6) and fibroblast growth factor 21 (FGF21), which are known to have a metabolism-improving action, have been reported as brown adipocyte-derived glucose metabolism-improving hormones, but the main source for IL6 is natural immune cells such as macrophages and mast cells, and for FGF21 it is "cells other than brown adipocytes" in pancreas and such. Therefore, it is nothing more than a part of those cytokines being produced by brown adipocytes, and thus, it is not appropriate to call them as brown adipocyte-derived, glucose metabolism-improving hormones.

BAT recovered from the body of mice is inappropriate as a material for the search for BATokines, which may be a reason why "true BATokines" have not been identified. Mouse BAT highly expresses strong degrading enzymes such as chymotrypsin-based proteolytic enzymes, trypsin-based proteolytic enzymes, and nucleic acid-degrading enzyme Rnase1, which are digestive enzymes produced by exocrine pancreatic glands. Therefore, from the moment of extraction of BAT from a mouse body, the quality of brown adipocytes begins to deteriorate rapidly. Because of this, it is extremely difficult to obtain the hormone group produced by BAT in an intact state (NPL 18).

On the other hand, it is impossible from an ethical viewpoint to obtain BAT from a human body and use it for research. In addition, there are no technologies for maintaining and culturing mature brown adipocytes recovered from mouse BAT or human BAT, for amplification culture, and for cryopreservation. That is, it is impossible to identify "true BATokines" using a BAT specimen.

As described above, in order to obtain a sufficient amount of BATokine-comprising brown adipocyte culture supernatant that retains BATokine activity for clinical application aimed at improving metabolism, there is a need for a technology for preparing high-quality brown adipocytes in a stable and timely manner without conflicting with ethical issues, or without being attacked by proteolytic enzymes. Here, it is essential for "high-quality brown adipocytes" to exert the action of improving glucose metabolism independently of the heat-producing ability, and the action of improving glucose metabolism should be seen in the supernatant thereof.

### [Citation List]

### [Patent Literature]

[PTL 1] WO2012/147853

### [Non-Patent Literature]

[NPL 1] The GBD 2015 Obesity Collaborators, New England Journal of Medicine, Vol. 377, pp. 13-27, 2017
[NPL 2] Yu et al., Annals of Internal Medicine, Vol. 166, pp. 613-620, 2017
[NPL 3] Farrar et al., BMJ, Vol. 354, i4694, 2016
[NPL 4] Villamor et al., JAMA, Vol. 317, pp. 925-936, 2017
[NPL 5] Persson et al., BMJ, Vol. 357, p. J2563, 2017
[NPL 6] Wang et al., JAMA, Vol. 317, No. 2515-2523, 2017
[NPL 7] Bragg et al., JAMA, Vol. 317, pp. 280-289, 2017
[NPL 8] Navar et al., JAMA, Vol. 316, No. 2041-2043, 2016
[NPL 9] Alegre-Diaz et al., New England Journal of Medicine, Vol. 375, pp. 1961-1971, 2016
[NPL 10] Dabelea et al., JAMA, Vol. 317, pp. 825-835, 2017
[NPL 11] Snook et al., JAMA, Vol. 317, pp. 971-973, 2017
[NPL 12] Schauer et al., New England Journal of Medicine, Vol. 376, pp. 641-651, 2017
[NPL 13] Huang et al., BMJ, Vol. 355, i5953, 2016
[NPL 14] van Marken Lichtenbelt et al., New England Journal of Medicine, Vol. 360, pp. 1500-1508, 2009
[NPL 15] Cypess et al., New England Journal of Medicine, Vol. 360, pp. 15091-1517, 2009
[NPL 16] Virtanen et al., New England Journal of Medicine, Vol. 360, pp. 1518-1525, 2009
[NPL 17] Saito et al., Diabetes, Vol. 58, pp. 1526-1531, 2009
[NPL 18] Kobayashi et al., Medical Research Archive, Volume 4, 2016
[NPL 19] Nishio et al., Cell Metabolism, Vol. 16, pp. 394-406, 2012
[NPL 20] Yoneshiro et al., Obesity, Vol. 19, pp. 1755-1760, 2011
[NPL 21] Beijer et al., Oncol Rev, Volume 6, Section e11, 2012
[NPL 22] Carson et al., Exerc Sport Sci Rev, Vol. 38, pp. 168-176, 2010
[NPL 23] Galat et al., Stem Cell Research & Therapy, Vol. 8, Article number 67, 2017
[NPL 24] Bendall et al., Nature, Vol. 448, pp. 1015-1021, 2007

### [Summary of Invention]

### [Technical Problem]

An objective of the present invention is to provide a composition capable of improving metabolism, which comprises a supernatant of brown adipocytes. Another objective of the present invention is to provide a method of preparing a supernatant of brown adipocytes capable of improving metabolism without using a culture solution comprising amino acids, vitamins, high-concentration glucose, and the like. A further objective of the present invention is to provide a method of producing brown adipocytes derived from pluripotent stem cells, which are useful for preparing brown adipocytes themselves and their supernatants.

The present invention also has the objective of providing a technique for preparing a supernatant component (SUP) capable of improving metabolism and which is obtained by culturing brown adipocytes (BA), which are produced from pluripotent stem cells in a "minimum required medium" free of exogenous cytokines, in a buffer comprising only salts and low glucose.

Another objective of the present invention is to provide a mitochondrial concentrate comprising a supernatant of brown adipocytes.

A further objective of the present invention is to provide a technique for preparing a supernatant component (SUP) capable of promoting wound healing, including a supernatant of brown adipocytes.

### [Solution to Problem]

The present inventors have developed a technique for producing brown adipocytes from pluripotent stem cells (PTL 1), and it has been confirmed that the produced pluripotent stem cell-derived brown adipocytes themselves exert a glucose metabolism-improving action in transplanted animals (NPL 19). In addition, the glucose metabolism-improving action is exhibited by 16 hours after transplantation of pluripotent stem cell-derived brown adipocytes (hereinafter, brown adipocytes), which suggests that this is not a secondary action associated with weight loss as a result of calorie consumption through heat production, and is a direct action by the transplanted cells. Thus, pluripotent stem cell-derived brown adipocytes satisfy the conditions for being an effective material even in clinical use for the purpose of improving metabolism.

Furthermore, since pluripotent stem cells have infinite proliferation ability, brown adipocytes can be stably prepared at any time. In addition, since the entire process up to the preparation of brown adipocytes is carried out in the cell culture room, it is completely free from attacks from proteolytic enzyme groups, which inevitably occur in the process of tissue recovery from living bodies and isolation of target cells. Therefore, by applying the technique for producing brown adipocytes from pluripotent stem cells, it is possible to obtain high-quality brown adipocytes stably and at any time only using general-purpose cell culture techniques. However, it is unknown whether the supernatant of brown adipocytes has a physiological activity, and it is not known how to obtain an active supernatant.

Human BAT decreases or disappears with age. For example, the BAT positive rate in PET-CT decreases from the latter half of the 20s, and becomes less than 30% in the 40s and a little more than 10% in the 50s (NPL 20). However, people who do not gain weight in middle age and has no change in visceral fat mass are BAT-positive (NPL 20). From this, the pathological condition of obesity/overweightness, metabolic syndrome, and type 2 diabetes can be viewed as a "brown adipocyte deficiency". Namely, for metabolic disorders such as obesity/overweightness, metabolic syndrome, and type 2 diabetes, the therapy of replacing the function of deficient brown adipocytes could bring revolutionary development to internal medical treatment of type 2 diabetes, which currently has inferior outcome compared to obesity surgery. Additionally, it would be epoch-making if the effect can be exhibited by supplementing only the supernatant of brown adipocytes without supplementing the brown adipocytes themselves.

In order to confirm whether the supernatant of brown adipocytes has a pharmacological effect, the present inventors first differentiated brown adipocytes from pluripotent stem cells maintained and cultured on a feeder as done before, and then attempted to collect the culture supernatant. The brown adipocyte differentiation medium comprises various amino acids, vitamins, and lipids necessary for cell survival, as well as high concentrations of glucose (around 17.5 mM), and is added with a cytokine cocktail necessary for brown adipocyte differentiation (IL6, VEGF, Flt-3L, SCF, BMP7). In order to eliminate these components, the present inventors cultivated brown adipocytes in a buffer comprising only salts and a high-concentration of glucose (16.8 mM) (hereinafter referred to as salt-glucose buffer), collected the supernatant, and measured the activity.

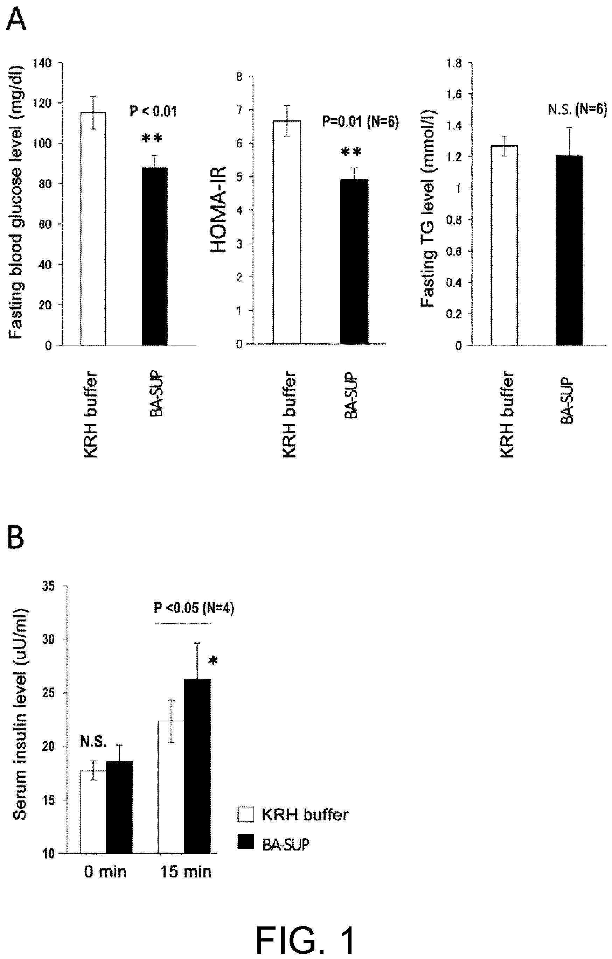

Compared to cell culture media, the salt-glucose buffer is overwhelmingly undernourished and does not comprise amino acids and protein components necessary for cell survival. Therefore, it was predicted that the supernatant collected by incubating brown adipocytes with a salt-glucose buffer would have almost no activity, but when this supernatant was subcutaneously administered to mice and the change in blood glucose level was measured, it was found that the fasting blood glucose level was significantly reduced (Fig. 1A, left). The HOMA-IR value, which is an insulin resistance index, was also significantly reduced by the administration of the supernatant (Fig. 1A, middle). Thus, it was found that the supernatant obtained by culturing brown adipocytes in a salt-glucose buffer has the activity of increasing insulin sensitivity and lowering the blood glucose level.

On the other hand, although the fasting triglyceride (TG) level is decreased in mice transplanted with pluripotent stem cell-derived brown adipocytes, the fasting TG level was not significantly decreased in the mice administered with the supernatant. Therefore, this suggests that the supernatant of brown adipocytes has a physiological activity that exerts an action of improving glucose metabolism, while the action of improving lipid metabolism is an action of brown adipocytes rather than the supernatant.

As described above, it was found that the supernatant of brown adipocytes exerts an action of improving glucose metabolism independently of body heat production by the fat burning of brown adipocytes. In addition, since the blood insulin level was increased in the supernatant-administered mice 15 minutes after the oral glucose tolerance test (Fig. 1B), it was suggested that the supernatant of brown adipocytes not only enhances insulin sensitivity, but also has the action of promoting insulin secretion.

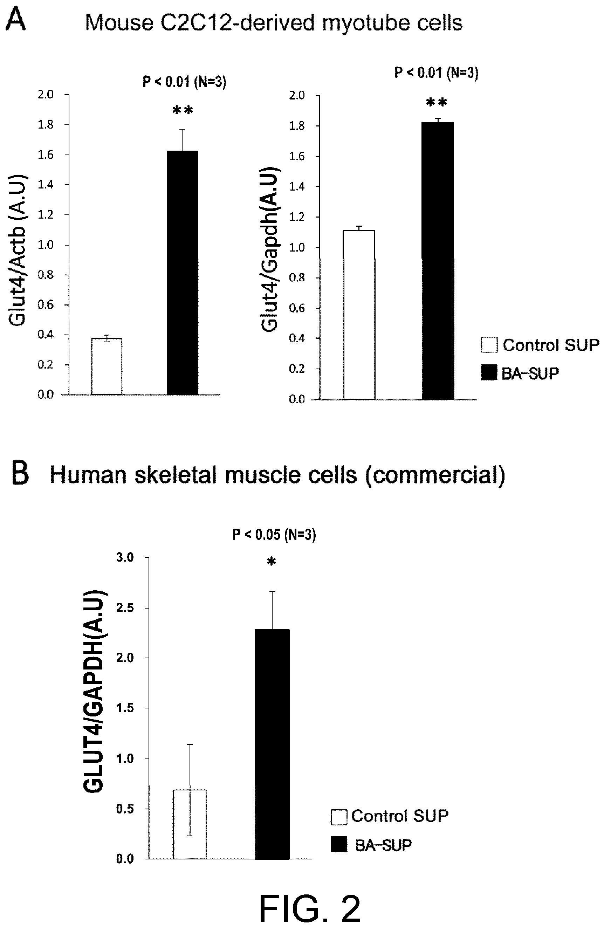

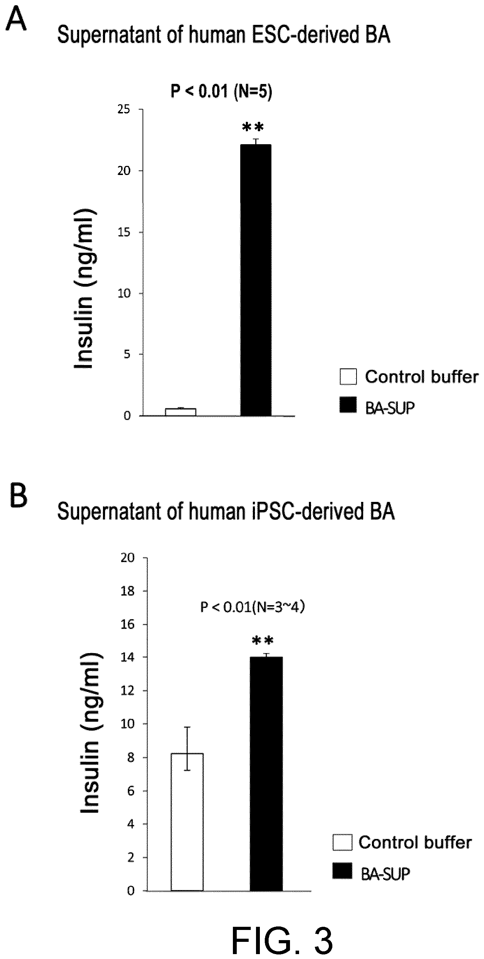

In addition, when the action of the supernatant on the glucose transporter gene Glut4 was examined in multiple skeletal muscle cells, it was found that the supernatant of brown adipocytes significantly increased the expression level of Glut4 (Figs. 2A and 2B). In addition, when the action of the supernatant on pancreatic beta cells was examined, it was found that the addition of the supernatant significantly increased the amount of insulin secreted (Figs. 3A and 3B).

Thus, it was shown that the supernatant of brown adipocytes has multiple actions such as lowering blood glucose level, increasing insulin sensitivity and promoting insulin secretion from pancreatic beta cells, and increasing the expression of glucose transporter gene in skeletal muscle cells, and that it is possible to obtain an active supernatant using a salt-glucose buffer that does not comprise nutritional components such as amino acids and vitamins.

Next, the present inventors tested whether an active supernatant could be obtained even with a salt buffer that does not comprise a high-concentration of glucose. Brown adipocytes were cultured in a salt buffer comprising a low concentration of glucose (2.8 mM) with the glucose concentration in the buffer reduced to 1/6, and the obtained supernatant was used to examine the insulin secretion-promoting action on pancreatic beta cells, which showed that insulin secretion was significantly increased, indicating that an active supernatant can be obtained from brown adipocytes even with low concentration-glucose buffers.

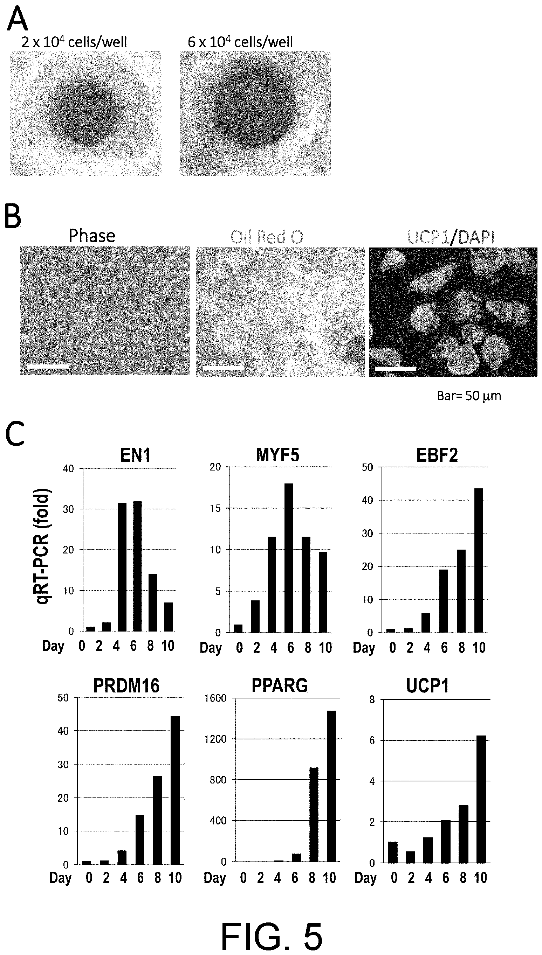

Brown adipocytes derived from pluripotent stem cells are differentiated from pluripotent stem cells that are maintained and cultured on feeder cells. In order to completely eliminate the influences from feeder cells, the inventors decided to culture the pluripotent stem cells in a feeder-free culture system and generate brown adipocytes from these cells to obtain a supernatant. Therefore, pluripotent stem cells were cultured in a feeder-free system, the obtained pluripotent stem cells were dispersed into single cells, and then spheres were formed by a suspension culture using a medium for producing cell aggregates, and the obtained spheres were collected and cultured in brown adipocyte induction medium. As a result, multilocular lipid droplets characteristic of brown adipocytes were observed in the cells (Fig. 5B left), which could be stained with Oil Red O (Fig. 5B), and the expression of the UCP1 protein involved in the thermogenetic ability of brown adipocytes (Fig. 5B right), as well as the expression of other marker genes for brown adipocytes were observed. Thus, it was found that brown adipocytes can be produced even from pluripotent stem cells cultured in a feeder-free culture system.

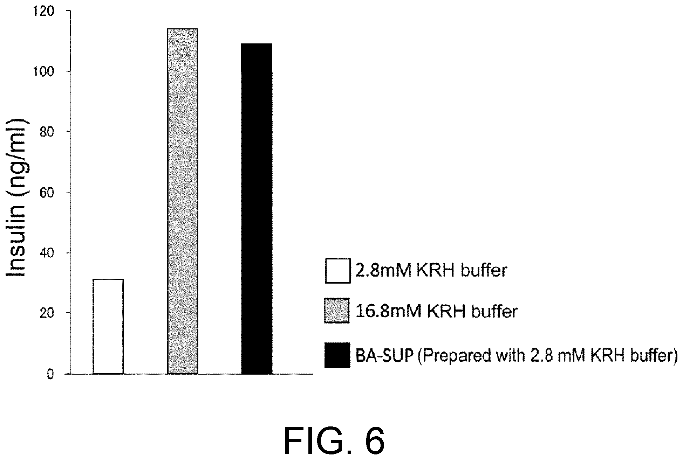

When the activity of the supernatant recovered by culturing, in a salt buffer comprising low-concentration glucose (2.8 mM) buffer, brown adipocytes generated from pluripotent stem cells cultured in a feeder-free culture system was examined, it was revealed that the supernatant exerts an insulin secretion-promoting ability as strong as when stimulated with high-concentration insulin (Fig. 6). Thus, the present inventors have succeeded for the first time in obtaining a supernatant of brown adipocytes that does not comprise any components derived from feeder cells and has an insulin secretion-promoting action, and the like. The supernatant of brown adipocytes can be desalted and can also be prepared as a lyophilized product while maintaining its activity.

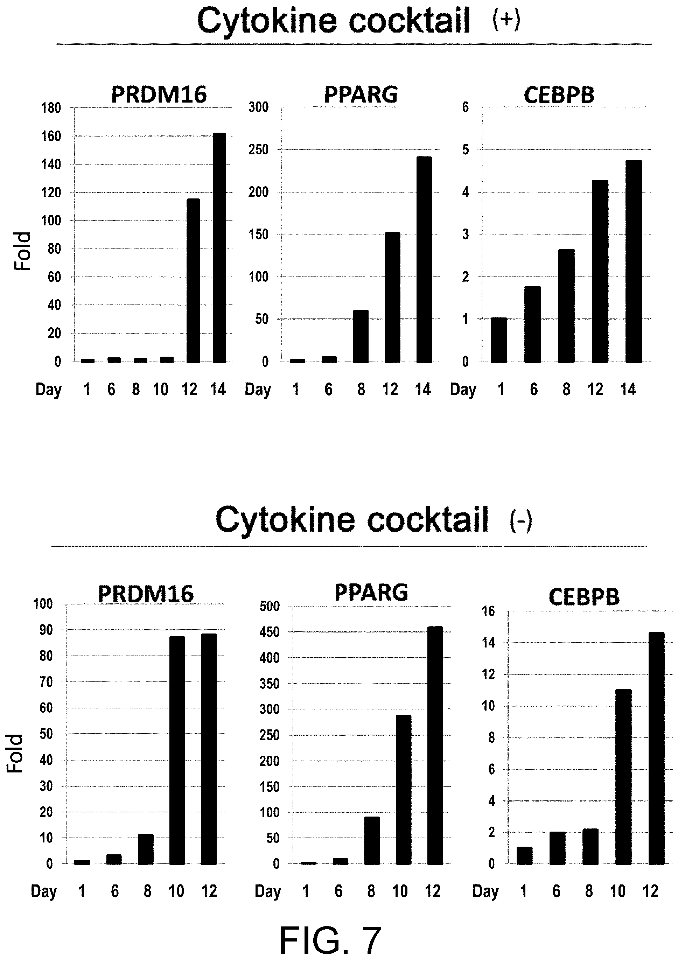

In addition, in the process of carrying out the above experiment to generate brown adipocytes using pluripotent stem cells cultured in a feeder-free culture system, the present inventors surprisingly discovered that, in pluripotent stem cells cultured with a feeder-free system, it was possible to spontaneously differentiate brown adipocytes just by forming spheres through a suspension culture (where the spheres are formed after pluripotent stem cells are dispersed into single cells and suspension cultured using a medium for producing cell aggregates comprising no cytokine cocktail) without adding a cytokine cocktail that was conventionally added to generate brown adipocytes, and then culturing the obtained sphere cells in a brown adipocyte induction medium comprising no cytokine cocktail (Fig. 7).

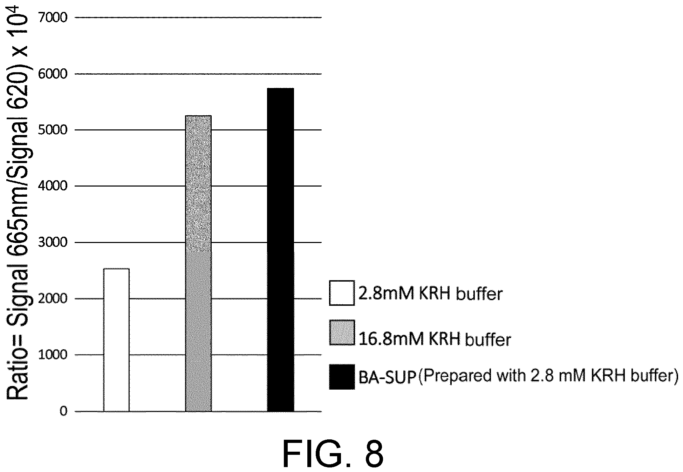

The supernatant obtained by culturing the brown adipocytes generated as above in a salt buffer comprising a low-concentration of glucose (2.8 mM) exhibited a strong insulin secretion-promoting ability. As a result, the present inventors succeeded, for the first time, in generating brown adipocytes from pluripotent stem cells without using a cytokine cocktail and also succeeded in obtaining a supernatant of those cells, which has the ability to promote insulin secretion (Fig. 8). Since this method does not use feeder cells and does not require the addition of cytokine cocktails, it is possible to produce extremely high quality brown adipocytes and their supernatants that do not comprise heterologous cells (cells other than brown adipocytes and the cells from which the brown adipocytes were derived) and do not comprise exogenous cytokines (cytokines that are not derived from pluripotent stem cells and brown adipocytes derived from them),.

The medium used to generate brown adipocytes from pluripotent stem cells is a serum-free medium, but includes bovine serum albumin (BSA), α-monothioglycerol (α-MTG), and protein free hybridoma mix (PFHMII, Gibco # 12040-077, Life Technologies, Inc.), which are said to have the effect of ensuring cell viability. In order to investigate whether these are essential for the induction of brown adipocyte differentiation, pluripotent stem cells maintained in a feeder-free culture system were induced to differentiate into brown adipocytes via a single cell dispersion operation in the same manner as above, and culturing was done using a medium comprising no cytokine cocktail and also removed of BSA, α-MTG, and PFHMII (minimum required medium). As a result, cell aggregates were formed as in the case of using a cytokine-comprising medium, and a cell morphology characteristic of mature brown adipocytes such as multilocular lipid droplets was observed, and marker gene expression specific to brown adipocytes was confirmed (Figs. 9A-F).

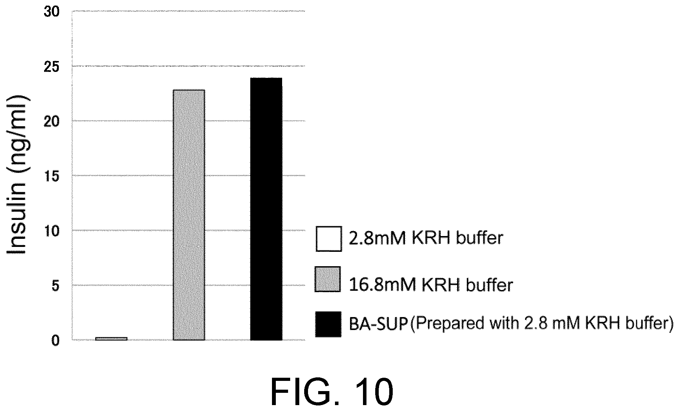

In order to obtain a supernatant of brown adipocytes prepared in a minimum required medium as described above from pluripotent stem cells maintained in a feeder-free culture system, the cells were cultured in a salt buffer comprising a low-concentration of glucose (2.8 mM), and the supernatant was collected. The brown adipocyte supernatant thus prepared also showed insulin secretion-promoting activity (Fig. 10).

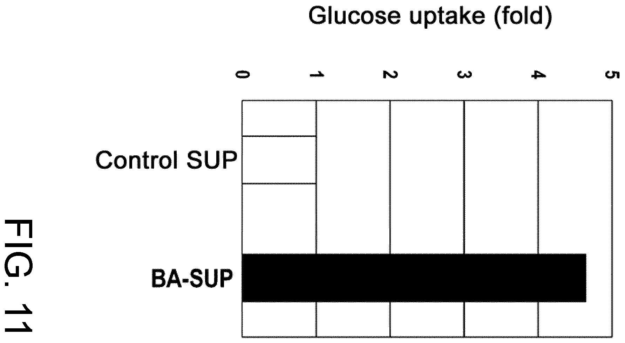

In addition, a supernatant was prepared , using a salt buffer comprising low-concentration glucose (2.8 mM), from brown adipocytes prepared as above in a minimum required medium from pluripotent stem cells maintained in a feeder-free culture system. When the influence on glucose uptake after adding this supernatant to pancreatic β cells was investigated, it was found that the addition of the supernatant significantly enhanced the glucose uptake ability of β cells (Fig. 11).

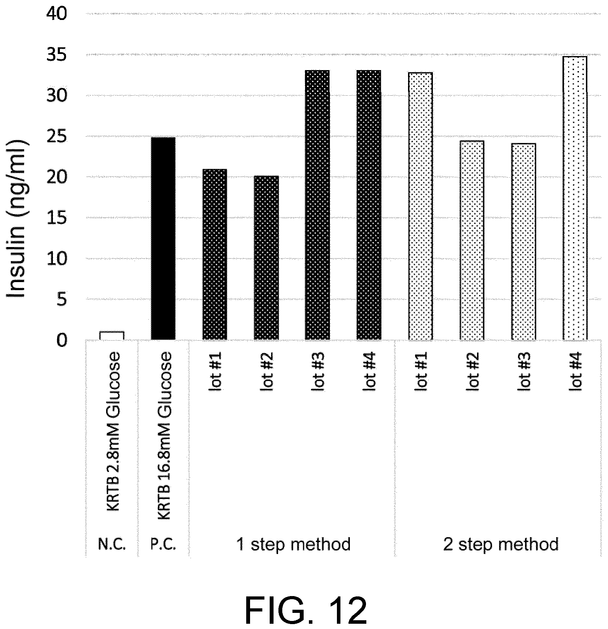

When a cytokine cocktail is not used, it is alright to use a medium having the same composition (for example, minimum required medium) for the medium for producing cell aggregates and the medium for inducing brown adipocytes. After dispersing the pluripotent stem cells maintained in the feeder-free culture system into single cells, a rotation culture using a bioreactor was performed in a minimum required medium, and brown adipocytes were produced in a one-step culture. The culture supernatant of the obtained cells showed an equivalent insulin secretion-promoting activity as the supernatant of brown adipocytes obtained in the two-step culture (Fig. 12).

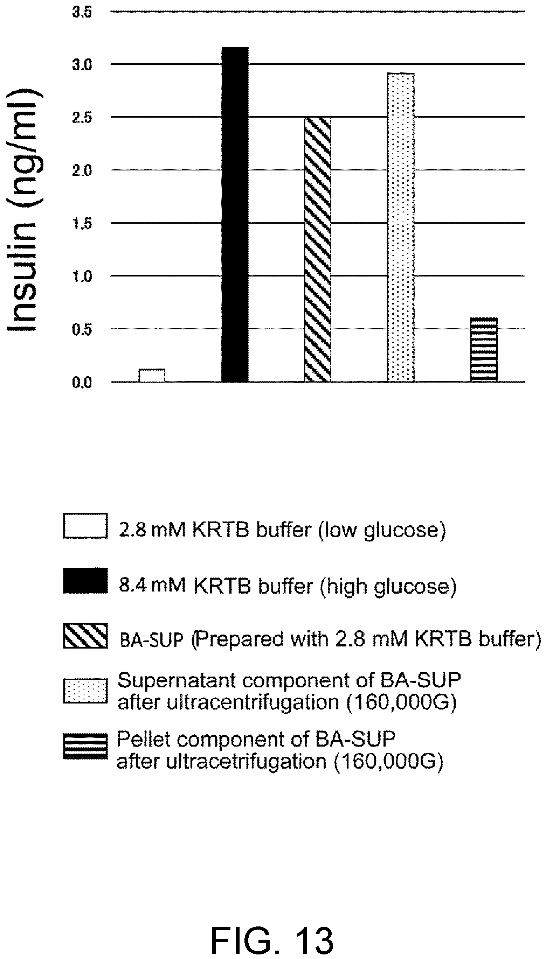

In addition, the supernatant of brown adipocytes was subjected to ultracentrifugation at 160,000 G, the supernatant component and sediment component (pellet) were separately collected, and the influence on insulin secretion was investigated using pancreatic β cells. Most of the insulin secretion-promoting activity was recovered in the supernatant component, but the insulin secretion-promoting activity was also observed in the pellet component (exosome fraction), albeit in a very small amount (Fig. 13). This suggests that the action of insulin secretion-promoting activity in the supernatant of brown adipocytes is mainly due to soluble factors, but it also includes exosomic factors.

In addition, it was revealed that the supernatant obtained by culturing brown adipocytes in a salt buffer comprising low-concentration glucose (2.8 mM) exerts an action of promoting glucose uptake by human skeletal muscle cells (Fig. 15), and that the action did not change even when the supernatant of brown adipocytes was subjected to biotinylation treatment (Fig. 16). It was also found that the culture supernatant of brown adipocytes promotes the glucose uptake ability of human cardiomyocytes (Fig. 17).

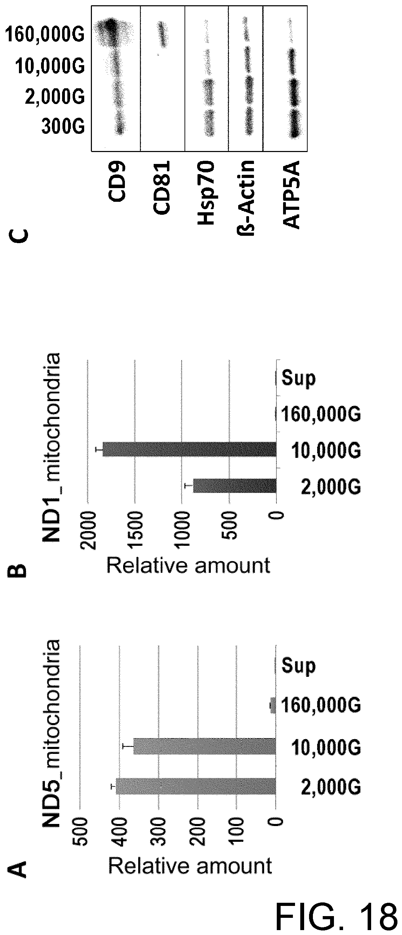

In addition, it was revealed that mitochondria are present in a concentrated manner in the precipitate collected by centrifuging the supernatant obtained by culturing brown adipocytes in a salt buffer comprising low-concentration glucose (2.8 mM) (Fig. 18).

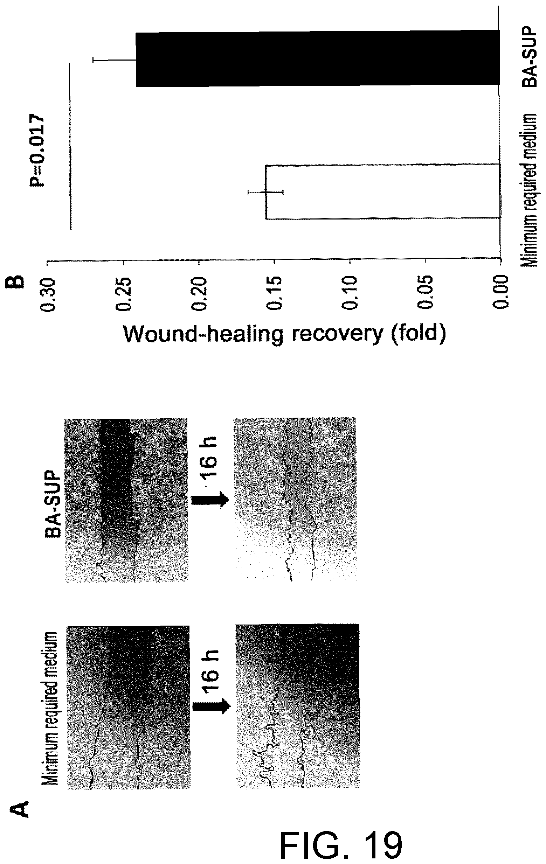

In addition, the supernatant obtained by culturing brown adipocytes in a minimum required medium was found to have an action of promoting repair from injury in a wound-healing assay using human vascular endothelial cells (Fig.19).

Since brown adipocytes comprise a large number of mitochondria with a high respiratory function, they are an excellent source of highly functional mitochondria. Mitochondrial transplantation therapy is very effective for severe pediatric heart failure where it is not possible to disengage from extracorporeal membrane oxygenator due to mitochondrial respiratory dysfunction (Emani et al., The Journal of Thoracic and Cardiovascular Surgery, Vol. 154, Pp. 286-289, 2017). The effectiveness of mitochondrial transplantation therapy for neonatal heart failure cases has also been reported (Gina Kolata, The New York Times, July 10, 2018, https://www.nytimes.com/2018/07/10/health/mitochondria-transplant-heart-attack.html). However, mitochondria prepared from autologous tissues such as autologous muscles are used for transplantation at present, and this imposes a time constraint of having to complete the procedure from tissue recovery to transplantation within 30 minutes in the operating room. Since the present invention provides a means that enables supplying of mitochondria having high respiratory function stably at any time without violating ethical issues, it is expected to contribute to the spread of mitochondrial transplantation therapy.

In addition, since mitochondria comprise a large amount of antioxidant proteins that have an eliminating action on active oxygen, it can be envisaged that antioxidant proteins may be present in the culture supernatant of brown adipocytes comprising a large amount of highly functional mitochondria. Since the concentration of active oxygen increases when tissue is damaged, it is assumed that the culture supernatant of brown adipocytes may promote recovery from damage to tissues such as skin through the action of eliminating active oxygen. The damage recovery-promoting action exhibited by the culture supernatant of brown adipocytes of the present invention may be due to the contribution of the active oxygen-eliminating action of mitochondria contained in the supernatant.

As described above, the present inventors have succeeded for the first time in obtaining a supernatant having an action of improving glucose metabolism using brown adipocytes produced from pluripotent stem cells. It was also found that it is not necessary to use a medium to obtain the supernatant, and an active supernatant can be obtained by culturing cells in a salt-glucose buffer comprising no amino acids or vitamins. The present invention also provides, for the first time, brown adipocytes produced using pluripotent stem cells cultured in a feeder-free culture system and a supernatant thereof. The present invention also provides, for the first time, a technique for producing brown adipocytes from pluripotent stem cells without adding a cytokine cocktail by using pluripotent stem cells cultured in a feeder-free culture system. When pluripotent stem cells cultured in a feeder-free culture system were used, not only was no cytokine cocktail required, but additives such as BSA, α-MTG, and PFHMII were not required as well. This makes it possible to obtain a supernatant component of brown adipocytes, in which the influence of heterologous cells and contamination with additives is little, and would also be advantageous when preparing as a preparation suitable for clinical application.

The supernatant of the present invention exhibits a remarkable insulin secretion-promoting action and glucose uptake-enhancing action on pancreatic β cells, significantly lowers the blood glucose level *in vivo,* and also significantly lowers the insulin resistance index (HOMA-IR value). In addition, since the supernatant of the present invention significantly increases the expression level of glucose transporter gene in skeletal muscle cells, muscle cells such as cardiomyocytes, etc., and exhibits an action of enhancing glucose uptake, it is expected to act on multiple organs and exert the action of improving glucose metabolism.

In addition, the supernatant of the present invention is expected to provide an excellent material for mitochondrial transplantation therapy, because the precipitate recovered by centrifugation, that is, the fine particle fraction, comprises concentrated mitochondria, and because the mitochondria of brown adipocytes have a high respiration capacity.

In addition, the supernatant of the present invention is expected to provide an excellent material for promoting healing of skin injury accompanied by vascular injury, because it was found to have an effect of promoting recovery from injury by a wound-healing assay using human vascular endothelial cells.

In other words, the present invention is defined in the claims. That is, the present invention encompasses the following:
[1] A method of producing a supernatant of brown adipocytes or brown adipocytes that can be used for producing the composition, comprising the steps of:
   (1) maintaining and culturing pluripotent stem cells feeder-free,
   (2) dispersing the cells of step (1) and culturing them non-adhesively in a serum-free environment without adding a cytokine cocktail comprising BMP4, VEGF, SCF, Flt3L, IL6, and IGF2 to form cell aggregates, and
   (3) culturing the cells of step (2) in a serum-free environment without adding a cytokine cocktail comprising BMP7, VEGF, SCF, Flt3L, IL6, and IGF2.
[2] The method according to [1], further comprising the step of:
   (4) incubating the brown adipocytes of step (3) in a salt buffer and collecting the supernatant thereof.
[3] The method according to [2], wherein the salt buffer is selected from Krebs- Ringer-Hepes (KRH), Krebs-Ringer-Tris (KRT), Krebs-Ringer Bicarbonate, Krebs-Ringer- Hepes-Bicarbonate (KRHB) or Krebs-Ringer-Tris Bicarbonate (KRTB).
[5] The method according to any one of [1] to [4], wherein in steps (2) and/or (3) the culturing is carried out without an exogenous cytokine.
[6] The method according to any one of [1] to [5], wherein steps (2) and (3) are carried out with a medium of identical composition in the same culture vessel.
[7] The method according to any one of [1] to [6], further comprising the step of desalting the obtained supernatant.
[8] A composition produced by the method according to any one of [1] to [7].
[9] The composition according to [8], wherein the composition does not comprise a cytokine cocktail comprising BMP7, VEGF, SCF, Flt3L, IL6, and IGF2.
[10] The composition according to [8] or [9], wherein the composition does not comprise an exogenous cytokine.
[11] The composition comprising the supernatant according to [8], wherein the composition is for use in the treatment of a metabolic disease or a metabolic disorder, preferably wherein the metabolic disease or metabolic disorder is selected from the group consisting of obesity, overweightness, metabolic syndrome, and type 2 diabetes; a disease caused by a mitochondrial dysfunction, preferably wherein the mitochondrial dysfunction is selected from the group consisting of mitochondrial disease and neonatal heart disease, or a disease caused by skin damage, preferably wherein the skin damage is selected from the group consisting of subcutaneous bleeding, pigmentation due to subcutaneous bleeding, and subcutaneous telangiectasia.

Cytokines such as IL6 and FGF2 have been analyzed as regulators of glucose metabolism and muscle mass, while it has been proposed that lipid mediators such as 12,13-diHOME, microRNAs such as miRNA-99b, and exosomes that carry them also be considered as regulators in a broad sense.

Thus, in addition to proteins such as cytokines, ensembles of various physiologically active substances including various molecule species including lipids and nucleic acids, and even extracellular fine particles could be one possibility of BATokines produced in brown adipocytes. In other words, in the advancement of the development of treatments for diseases of metabolic disorders focusing on brown adipocytes, prior to the search for BATokines and the elucidation of the mechanism of action, an important task is the development of technologies for preparing "BATokines as an ensemble of various bioactive substances" which have a metabolism-improving action.

As shown in the present invention, the supernatant of brown adipocytes can be an ideal material for preparing BATokines as an "ensemble of various bioactive substances". However, further analysis and research were needed to determine whether the supernatant of brown adipocytes had an advantageous physiological activity and what kind of buffer should be used in preparing the supernatant. When considering clinical application, it is desirable to use a "buffer comprising only salts and glucose" (hereinafter referred to as "salt-glucose buffer"), but it is overwhelmingly less nutritious than cell culture media, and it was unclear whether a supernatant collected by culturing brown adipocytes in a salt-glucose buffer comprising no amino acid or protein component necessary for cell survival would indeed have metabolism-improving action

In addition, the brown adipocyte differentiation-inducing medium for pluripotent stem cells comprises a high concentration (> 17 mM) of glucose (see Reference Example 4). Due to this, it was unclear whether a supernatant prepared with a normal salt-glucose buffer comprising a low-concentration of glucose would have a metabolism-improving action. However, when considering clinical application for the treatment of diseases of metabolic disorders, it is not preferable to use a buffer comprising a high concentration of glucose.

Another issue was that a cocktail comprising many types of synthetic cytokines (a synthetic cytokine cocktail) is added to the differentiation-inducing medium used when producing brown adipocytes from pluripotent stem cells (see Reference Example 4). The use of exogenous cytokines such as synthetic cytokines (cytokines added to cells, or cytokines expressed by introducing cytokine genes into cells) is costly and complicated in terms of time and effort required for quality control and such. In addition, this cocktail comprises IL6, which causes cachexia, and VEGF, which promotes the progression of "cancer" because it has an angiogenic potential, and for clinical application, they should be mixed at the lowest level possible. The best way to do this was to develop a technique for producing brown adipocytes from pluripotent stem cells without the use of synthetic cytokine cocktails.

Since the invention of human-induced pluripotent stem cells (iPS cells), research toward the development of therapeutic techniques using human iPS cells and their supernatants has been accelerating. While many of the conventional differentiation induction methods use synthetic cytokine cocktails, there is a need to develop a differentiation induction technology that does not use synthetic cytokine cocktails, that is, a technology that uses an "exogenous cytokine-free medium". However, to date, there has been only one report of successful differentiation induction of human pluripotent stem cells using an "exogenous cytokine-free medium", which was a technique for inducing differentiation of hemogenic endothelium from human iPS cells in an "exogenous cytokine-free medium" reported in 2017 (NPL 23). Here, as a substitute for exogenous cytokines, the low molecular weight compound CHIR99021 was added, which is an agonist of WNT, a protein having a strong cell differentiation regulatory action. However, when clinically applying an extract or a culture supernatant of differentiated cells derived from human pluripotent stem cells, the application of a differentiation induction system using a medium supplemented with a low molecular weight compound having a specific physiological action cannot be said to be a favorable differentiation-inducing condition considering the side effects caused by the contamination of low molecular weight compounds.

Furthermore, in NPL 23 in the preceding paragraph, the undifferentiated maintenance culture of human iPS cells was done in a co-culture system with mouse fetal fibroblasts (MEF). However, MEF is known to have a strong "differentiation induction inhibitory action". The mechanism is believed to be that MEF secretes FGF2, which is also added in the feeder-free maintenance culture system of human pluripotent stem cells, and this acts as a paracrine factor on human pluripotent stem cells (NPL 24). In the differentiation induction of human pluripotent stem cells that have been maintained and cultured undifferentiated in a co-culture system with MEF, it has been reported that a considerable amount of MEF is mixed in the "initial step of differentiation induction" such as embryoid body formation. Moreover, since MEF exists in contact with human pluripotent stem cells, FGF2 produced by the contaminating MEF acts on human pluripotent stem cells at a high concentration, and as a result, differentiation induction is strongly inhibited. Therefore, in order to overcome the inhibitory action on differentiation induction by the mixed MEF and pursue differentiation induction, a cytokine cocktail or a low-molecular-weight compound which is a strong driver of differentiation induction is required. It is considered that this is also why cytokines, small molecule agonists, and the like are added as such "strong differentiation induction drivers" in conventional differentiation induction systems.

For clinical application of human pluripotent stem cell-derived differentiated cells and their extracts or culture supernatants, simplifying the differentiation induction system by using a medium comprising no exogenous cytokines or low molecular weight compounds is not only cost-effective, but is also important from the viewpoint of securing safety. For this purpose, utilization of signals of "autocrine/paracrine factors secreted by human pluripotent stem cells themselves" as much as possible rather than utilizing exogenous cytokines and small molecule compounds and thereby establishing a highly-oriented differentiation induction system becomes the key. For this, a first indispensable step is to completely eliminate the contamination of feeder cells (MEF) from the differentiation induction system, that is, to make the undifferentiated maintenance culture a feeder-free culture. The present invention has not only realized feeder-free production of brown adipocytes, but also established a technique for autonomously differentiating brown adipocytes from pluripotent stem cells without using exogenous cytokines or the like.

In medium development, it rarely turns out that components that were previously thought to be essential are found to be non-essential. For example, the Essential 8 Medium (Thermo Fisher Scientific), a general-purpose feeder-free medium for human pluripotent stem cells, was developed by identifying "minimal requirements" from the preceding mTeSR1 feeder-free medium (Stem Cell Technologies). Bovine serum albumin (BSA), which was considered essential until then, was only needed to cancel the toxicity of the reducing agent betamercaptoethanol (b-ME), and it was found that BSA itself it is not essential for maintaining the undifferentiated state of human pluripotent stem cells. Furthermore, it was found that b-ME, which was added to various media for the purpose of preventing natural oxidation by air, is not necessary for maintaining undifferentiated human pluripotent stem cells. In other words, BSA is an essential element under conditions where b-ME is added, but is non-essential under conditions where b-ME is not present. The differentiation of brown adipocytes found in the present invention can also be regarded as the discovery of one of such rare events.

In the differentiation induction of pluripotent stem cells of the present invention, one reason for succeeding in eliminating the need for adding exogenous cytokines and obtaining high-quality pluripotent stem cell-derived differentiated cells and their supernatants could be that, through the culture system of the present invention becoming a condition that kills cells that differentiate into a cell lineage other than the target lineage, a high differentiation orientation towards brown adipocytes was imparted.

For example, in Reference Example 4, which is a technique for inducing differentiation of brown adipocytes from pluripotent stem cells maintained and cultured with feeder cells (MEF), a cell aggregate formation step (step (A)) is performed as the first step. In co-culture systems with MEF, pluripotent stem cells are treated as "monolayer cell crops", so if undifferentiated pluripotent stem cells are detached from the culture dish and suspended in culture, "spherical cell crops (spheres)" are easily formed. On the other hand, pluripotent stem cells maintained in a feeder-free culture system are often dispersed at the single cell level after detaching from the culture dish. It is not always easy to reshape cell aggregates from the state of being dispersed into single cells, and specific elements (nutrients, cytokines, etc.) are required for producing "spherical cell crops (spheres)" using single cells, and as a result, it is speculated that only cells that can differentiate into brown adipocytes selectively form spheres.

When comprehensively considering the above, it can be postulated that the production of brown adipocytes under the condition of no feeder/no cytokine addition was successful due to the two conditions of: (1) making the undifferentiated maintenance culture a feeder-free culture and making the best use of the autocrine/paracrine factors secreted by pluripotent stem cells obtained in association with it, and (2) discovering the conditions where cells that can differentiate into lineages other than brown adipocytes are actively killed or eliminated in the process of cell crop (sphere) formation by purposely not adding a cytokine cocktail used in the conventional method (Reference Example 4).

As described above, the present invention has succeeded in providing a new technique capable of producing brown adipocytes suitable for clinical application and their supernatants from pluripotent stem cells. The cells and supernatants of the present invention have an insulin secretion-promoting action, a glucose uptake-enhancing action of pancreatic beta cells, skeletal muscle cells, and myocardial cells, and an effect of improving glucose metabolism. By administering the supernatant of the present invention or a purified product or such thereof, it becomes possible to replenish various BATokines deficient in diseases of metabolic disorders such as obesity/overweightness, metabolic syndrome, and type 2 diabetes, in a well-balanced and safe manner. That is, a new internal medical treatment for diseases of metabolic disorders using brown adipocytes or their supernatants was developed.

Further, since the brown adipocytes and supernatants of the present invention can comprise a large number of mitochondria, they are also useful for prevention or treatment of various diseases presenting a mitochondrial dysfunction such as mitochondrial disease and neonatal heart disease. Further, since the brown adipocytes and supernatants of the present invention have an action of promoting injury healing, they are also useful in prevention or treatment for various diseases showing skin damage such as subcutaneous bleeding, pigmentation due to subcutaneous bleeding, and subcutaneous telangiectasia.

As to the preparation of brown adipocytes and their supernatants, for example, brown adipocytes are prepared from pluripotent stem cells according to the method of the present invention, and in the case of collecting the supernatant, for example, brown adipocytes are cultured in a salt-glucose buffer, the supernatant is collected, and the effect of improving metabolism is evaluated. At this time, since the salt-glucose buffer is poor in nutritional components, the viability of brown adipocytes is reduced in long-term cultures, and the metabolism-improving action of the supernatant may be reduced. On the other hand, it is not possible to prepare a supernatant having sufficient activity by culturing for a short time. Therefore, the basic composition (including glucose concentration) of the salt-glucose buffer and the optimum culture time for recovering a supernatant having a metabolism-improving action are determined.

For example, the culture conditions for producing brown adipocytes from pluripotent stem cells using a differentiation-inducing medium not added with a synthetic cytokine cocktail are determined. Furthermore, the culture conditions for producing brown adipocytes from pluripotent stem cells using a medium from which additives for serum-free media have been removed, such as the PFHM-II Protein-Free Hybridoma Medium (GibcoTM), which does not comprise animal-derived proteins such as bovine serum albumin and cytokines, but comprises physiologically active molecules such as female hormones, are determined. Then, the pluripotent stem cell-derived brown adipocytes prepared using the determined "minimum required medium" are cultured in a salt-glucose buffer, and the supernatant is collected.

The brown adipocyte supernatant prepared thereby also comprises "brown adipocyte-derived fine particles (exosomes, etc.)" which is BATokine in a broad sense. In recent years, attention has been paid to the fact that fine particles secreted by cells, such as exosomes, exert various physiological actions. In particular, it has been reported that brown adipocytes are the main source of exosomal microRNAs present in the circulating blood, and that brown adipocyte-derived exosomal microRNAs contribute to improving metabolism (Thomou et al., Nature 542: 450-455, 2017). That is, the supernatant of brown adipocytes prepared in the present invention provides a suitable material for studies in search of BATokines in a broad sense, and clinical applications aimed at treating diseases presenting metabolic disorders such as obesity/overweightness, metabolic syndrome, type 2 diabetes, etc., using BATokine in a broad sense.

The supernatant of brown adipocytes prepared above is subjected to ultracentrifugation at an acceleration of 160,000 G, and the supernatant obtained when fine particles secreted by brown adipocytes are precipitated is collected. By this operation, extracellular fine particles such as exosomes contained in the supernatant are removed, and BATokine in a narrow sense is obtained. In cases where the administration of extracellular fine particles is judged to be unfavorable for clinical application for the treatment of diseases showing metabolic disorders such as obesity/overweightness, metabolic syndrome, and type 2 diabetes, this "BATokine in a narrow sense" provides a more appropriate material.

Further, the supernatant of brown adipocytes of the present invention is an effective material for basic research for searching for unidentified BATokine molecules and drug discovery research.

Moreover, the supernatant of brown adipocytes of the present invention is an effective material for studying extracellular fine particles produced by brown adipocytes.

Also, the supernatant of brown adipocytes of the present invention is an effective material for clinical application aiming at prevention or treatment of various diseases showing metabolic abnormalities such as obesity/overweightness, metabolic syndrome, and type 2 diabetes.

Further, the supernatant of brown adipocytes of the present invention is an effective material for clinical application aiming at prevention or treatment of various diseases showing mitochondrial dysfunction such as mitochondrial disease and neonatal heart disease.

Moreover, the supernatant of brown adipocytes of the present invention is an effective material for clinical application aiming at prevention or treatment of various diseases showing skin damage such as subcutaneous bleeding, pigmentation due to subcutaneous bleeding, and subcutaneous telangiectasia.

### [Effects of the Invention]

The present invention has discovered that the supernatant (BA-SUP) of brown adipocytes (BA) has a metabolism-improving action, and has provided BA-SUP and a method for producing it as novel tools for searching for BATokine, and also for the purpose of providing high-potency BA-SUP and a dried product and such derived from the BA-SUP, which can be therapeutic tools for diseases of metabolic disorders such as obesity and diabetes. Furthermore, the present invention has provided a method for producing BA without adding cytokines and a method for recovering BA-SUP from the cells. In particular, according to the present invention, it is also possible to prepare a desalted, metabolism-improving ability-retaining concentrate of a supernatant obtained using a buffer comprising only salts and low-concentration glucose and using BA prepared from pluripotent stem cells in a "minimum required medium" comprising no synthetic cytokines.

Since human pluripotent stem cells have an infinite proliferation ability and pluripotent differentiation ability, various human cells and tissues can be produced, but evaluation of the risk of cells becoming cancerous is important in transplantation therapy using the cells themselves. On the other hand, therapy by administration of a culture supernatant of human pluripotent stem cell-derived differentiated cells does not carry such a risk. However, contamination with animal-derived factors, which are often used in the differentiation process of human pluripotent stem cells, must be avoided in clinical use, and additives such as synthetic cytokines and low-molecular-weight compounds having specific pharmacological actions should also preferably be avoided as much as possible. Through the present invention, it is possible to induce differentiation of BA in a "minimum medium" from which these additives have been removed. For clinical use of a human pluripotent stem cell-derived differentiated cell supernatant, the development of a differentiation induction technology in a minimum medium, and the development of a technology for preparing a differentiated cell supernatant with a buffer comprising only salts and low-concentration glucose are necessary.

Since the supernatant (BA-SUP) prepared with a buffer comprising only salts and low-concentration glucose using the BA prepared by the method of the present invention has the action of promoting insulin secretion and enhancing insulin sensitivity (enhancing glucose uptake by insulin target cells), the possibility of BA-SUP administration therapy for diseases of metabolic disorders has been shown. Furthermore, BA-SUP can be prepared as a desalted concentrate or a dried solid product such as those through lyophilization. Such a highly active BA-SUP desalted concentrate is a material suitable for clinical application. In addition, as the BA-SUP of the present invention can be prepared from "feeder-free pluripotent stem cells" in a "minimum required medium" that does not comprise components such as synthetic cytokines, and using a buffer that comprises only salts and low-concentration glucose, GMP can be achieved by a general-purpose method, and development targeting clinical application is also possible.

Described above are new techniques useful for drug discovery research applying the glucose metabolism-improving action of a supernatant of the present invention, for development of new therapeutic tools for diseases of metabolic disorders, and the like.

Through the present invention, studies (including preclinical studies) using animals with the aim of developing new prevention/treatment for various diseases showing metabolic abnormalities such as obesity/overweightness, metabolic syndrome, type 2 diabetes, etc. using brown adipocytes and their supernatants will be accelerated.

Through the present invention, studies (including preclinical studies) using animals with the aim of developing new prevention/treatment for various diseases showing mitochondrial dysfunction such as mitochondrial disease and neonatal heart disease using brown adipocytes and their supernatants will be accelerated.

Through the present invention, studies (including preclinical studies) using animals with the aim of developing new prevention/treatment for various diseases showing skin damage such as subcutaneous bleeding, pigmentation due to subcutaneous bleeding, and subcutaneous telangiectasia using brown adipocytes and their supernatants will be accelerated.

Through the present invention, the development of new preventive/therapeutic methods for various diseases showing metabolic abnormalities such as obesity/overweightness, metabolic syndrome, and type 2 diabetes using brown adipocytes and their supernatants will be accelerated.

Through the present invention, the development of new preventive/therapeutic methods for various diseases showing mitochondrial dysfunction such as mitochondrial disease and neonatal heart disease using brown adipocytes and their supernatants will be accelerated

Through the present invention, the development of new preventive/therapeutic methods for various diseases showing skin damage such as subcutaneous bleeding, pigmentation due to subcutaneous bleeding, and subcutaneous telangiectasia using brown adipocytes and their supernatants will be accelerated

The brown adipocytes of the present invention and their supernatants are also useful for various cosmetic purposes.

### [Brief Description of Drawings]

[Fig. 1] The action of improving glucose tolerance of a brown adipocyte supernatant (BA-SUP) prepared with a "buffer comprising only salts and high-concentration glucose" when administered to mice. The BA-SUP was prepared by culturing brown adipocytes (BA) prepared from human ESC by the method described in Reference Example 4 in KRH buffer (comprising 16.8 mM glucose) for 16 hours. 500 ml of KRH buffer (comprising 16.8 mM glucose) or BA-SUP were administered subcutaneously to10-week-old ICR strain mice, and after 16 hours of fasting, the fasting blood glucose level, fasting serum insulin level, and fasting blood triglyceride level were measured (A). The HOMA-IR value, which is an index of insulin resistance, was calculated from the fasting blood glucose level and the fasting insulin level. In addition, a glucose solution was orally administered, and the blood glucose level and the serum insulin level were measured 15 minutes later (B).
[Fig. 2] Glut4/GLUT4 expression-inducing action of BA-SUP prepared in a "differentiation medium" on myotube cells. To mature skeletal muscle cells (myotube cells) induced to differentiate from mouse myoblast line C2C12 (A) or human skeletal muscle cells (B), a supernatant recovered by culturing the differentiation medium described in Reference Example 4 (for step (B)) in a gelatin-coated dish for 16 hours (Control SUP), or BA-SUP prepared by culturing human ESC-derived BA in a differentiation medium for 16 hours were added, RNAs were prepared from the cells 16 hours later, and the expression of Glut4 and GLUT4 genes was respectively measured by quantitative RT-PCR.
[Fig. 3] The insulin secretion-promoting action of BA-SUP prepared with "a buffer comprising only salts and high-concentration glucose" on pancreatic beta cells. BA-SUP prepared by culturing BA prepared from human ESC (KhES-3 strain) (A) or from human iPSC (derived from HUVEC, PTL 1, NPL 19) (B) in KRH buffer (comprising 16.8 mM glucose) for 16 hours, or KRH buffer (comprising 16.8 mM glucose), was added to MIN6 cells, and 2 hours later, the insulin concentration in the cell supernatant was measured by the ELISA method. In ELISA measurements, the insulin value (ng/ml) cannot be compared between different experiments because the sample is appropriately diluted and measured so as to fit in the quantitative region.

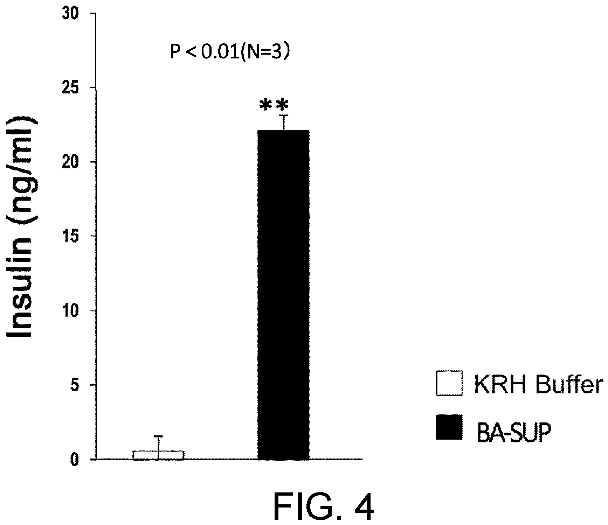

[Fig. 4] The insulin secretion-promoting action of BA-SUP prepared with "a buffer comprising only salts and low-concentration glucose" on pancreatic beta cells. BA-SUP prepared by culturing BA prepared from human ESC (KhES-3 strain) in KRH buffer (comprising 2.8 mM glucose) for 16 hours, or KRH buffer (comprising 2.8 mM glucose), was added to MIN6 cells, and after 2 hours, the insulin concentration in the cell supernatant was measured by the ELISA method.
[Fig. 5] BA differentiation induction technique for "human pluripotent stem cells maintained in a feeder-free culture system". Human ESCs (KhES-3 strain) maintained in a feeder-free culture system using Essential 8 medium and Matrigel^{™} Matrix coated dish were detached and collected, dispersed into single cells, and then a fixed number of cells were seeded onto a low-adsorption 96-well culture dish. This was cultured in the medium for step (A) of Reference Example 4 for 8 days to prepare cell aggregates (A). Then, BA was prepared by culturing in the medium for step (B) of Reference Example 4 for another 2 days (B). In addition, the expression induction status of BA-related genes was evaluated by quantitative RT-PCR (C). BA differentiation was induced comparable to the case of using the KhES-3 strain maintained on MEF described in Reference Example 4, and it was also confirmed that it is a "differentiation-inducing system that correctly reproduces the BA development process *in vivo"* via EN1-positive somite cells and MYF5-positive myoblasts.
[Fig. 6] Insulin secretion-promoting action on pancreatic beta cells of BA-SUP prepared with "a buffer comprising only salts and low-concentration glucose" using BA prepared from "feeder-free cultured human ESC". A human ESC (KhES-3 strain)-derived BA prepared in the same manner as in Fig. 5 using human ESC (KhES-3 strain) maintained in a feeder-free culture system in a StemFit^{™} AK02N and vitronectin-coated dish were cultured in KRH buffer (comprising 2.8 mM glucose) for 16 hours to obtain a BA-SUP. This BA-SUP or KRH buffer (comprising 2.8 mM glucose) was added to MIN6 cells, and 2 hours later, the insulin concentration in the cell supernatant was measured by the ELISA method. It can be seen that the addition of BA-SUP produces the same level of insulin secretion as a high glucose environment (equivalent to 300 mg/dL) despite being a low glucose environment (equivalent to 50 mg/dL).
[Fig. 7] Induction of BA differentiation of "feeder-free human pluripotent stem cells" using a "cytokine cocktail-free medium"
[Fig. 8] Insulin secretion-promoting action on pancreatic beta cells of BA-SUP prepared with "a buffer comprising only salts and low-concentration glucose" using BA prepared in a " medium comprising no cytokine cocktail" from "feeder-free cultured human ESCs". The vertical axis shows the fluorescence intensity based on the fluorescence resonance energy transfer (FRET) obtained as a proportional value of insulin concentration.

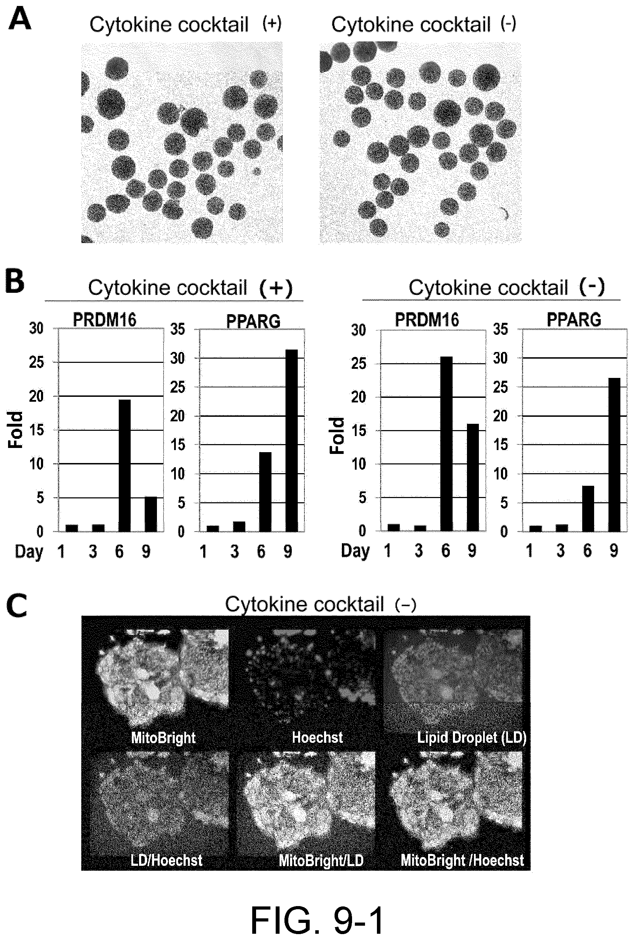

[Fig. 9-1] Induction of BA differentiation of "feeder-free human pluripotent stem cells" using a "minimum essential medium comprising no cytokine cocktail". A: A phase-contrast micrograph (objective lens 4x) of cell aggregates on day 3 of differentiation induction (KhES-3 strain). B: Changes in the expression of BA-related genes over time (GAPDH-corrected values) (KhES-3 strain). C: Cell morphology (mitochondria (green), lipid droplets (red), nuclei (blue)) (KhES-3 strain) on day 10 of differentiation induction in a medium comprising no cytokine cocktail.

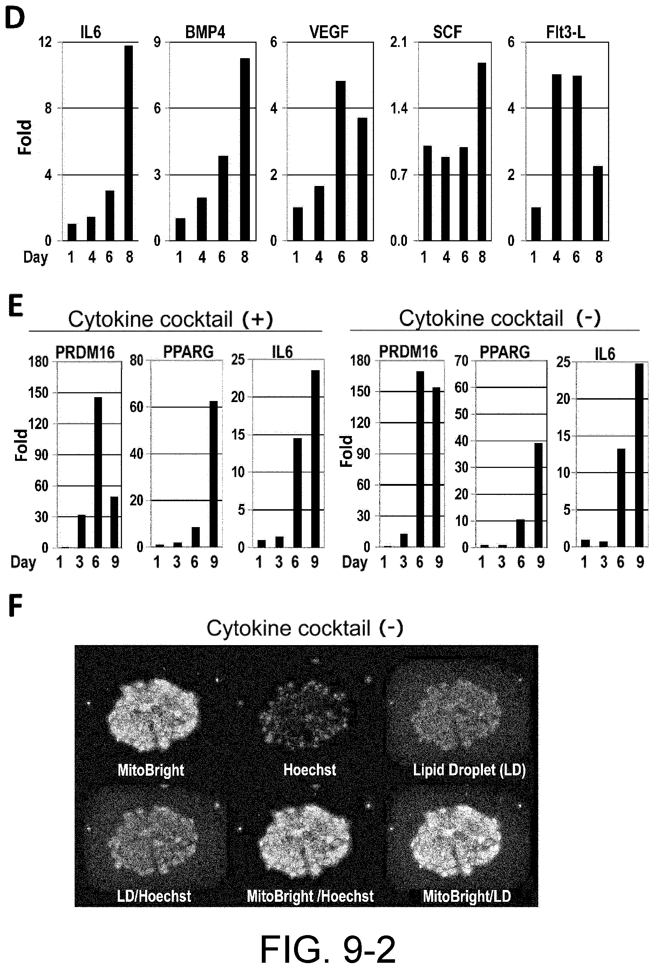

[Fig. 9-2] Induction of BA differentiation of "feeder-free human pluripotent stem cells" using a "minimum essential medium comprising no cytokine cocktail". D: Changes over time in the expression of endogenous genes encoding each of the cytokines that constitute the cytokine cocktail (KhES-3 strain). E: Changes in the expression of BA-related genes over time (GAPDH-corrected values) (KhES-1 strain). F: Cell morphology (mitochondria (green), lipid droplets (red), nuclei (blue)) (KhES-1 strain) on day 10 of differentiation induction in a medium comprising no cytokine cocktail.
[Fig. 10] The insulin secretion-promoting action on pancreatic beta cells of BA-SUP obtained using "a buffer comprising only salts and low-concentration glucose" and BA prepared from feeder-free human ESC in a minimum required medium.
[Fig. 11] The glucose uptake enhancing action on pancreatic beta cells of BA-SUP obtained using "a buffer comprising only salts and low-concentration glucose" and BA prepared from feeder-free human ESC in a minimum required medium.
[Fig. 12] The insulin secretion-promoting action of human BA supernatant obtained using "a buffer comprising only salts and low-concentration glucose" and BA prepared from "feeder-free human ESC" using a "minimum required medium" by "1 step" of suspension culture only.
[Fig. 13] Effect of ultracentrifugation on insulin secretion-promoting activity of BA-SUP obtained using a "buffer comprising only salts and low-concentration glucose" and BA prepared from feeder-free human ESC using a "minimum required medium".

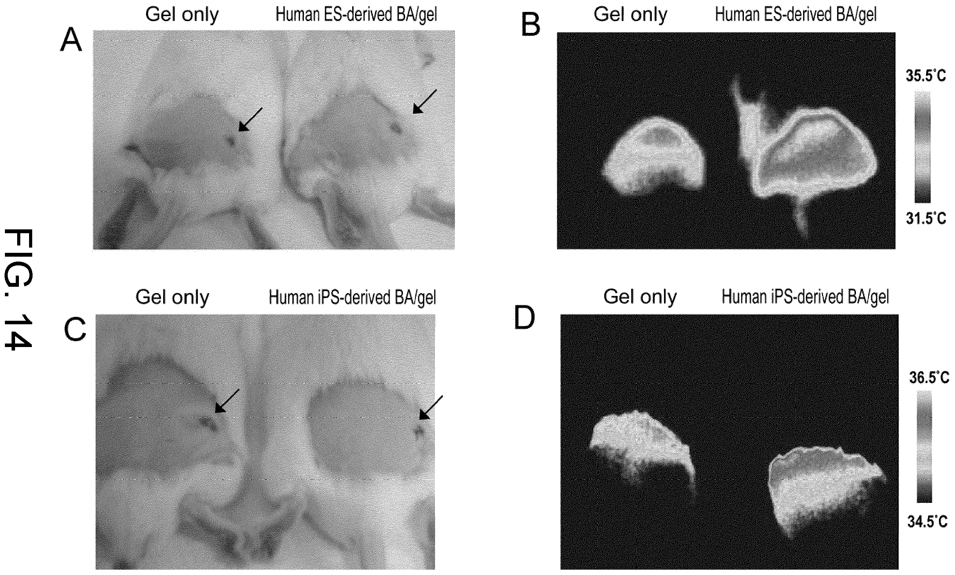

[Fig. 14] Heat producing ability of BA induced to differentiate in a minimum required medium (*in vivo* evaluation). A, C: A gel-only transplant (sham) or BA-embedded gel (BA transplant) was injected into skin incision wounds of mice whose buttocks have been depilated in advance. Pictures of mice taken 48 hours later. B, D: Infrared camera photographs taken 6 minutes after the administration of Isoproterenol.

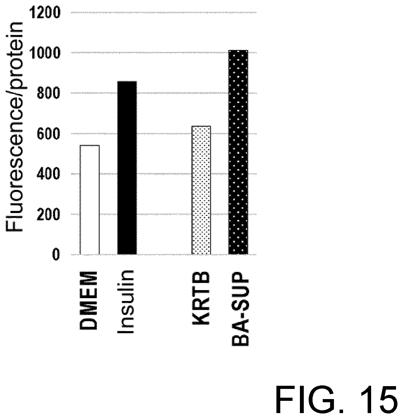

[Fig. 15] A graph showing the glucose uptake-promoting action on human skeletal muscle cells (SkMC) of BA-SUP obtained using "a buffer comprising only salts and low-concentration glucose" and BA prepared from feeder-free human ESC using a "minimum required medium".

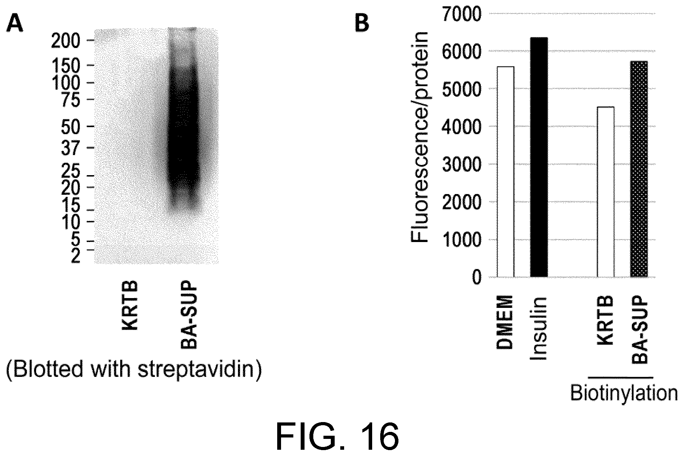

[Fig. 16] Figures showing the effect on the glucose uptake- promoting action on human skeletal muscle cells (SkMC) when BA-SUP was biotinylated, where the BA-SUP was obtained using "a buffer comprising only salts and low-concentration glucose" and BA prepared from feeder-free human ESC using a "minimum required medium".

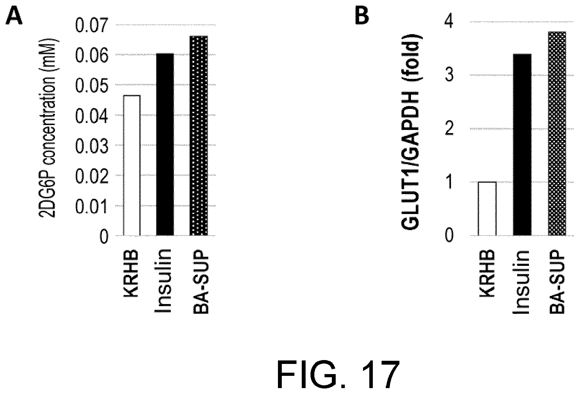

[Fig. 17] Graphs showing the glucose uptake-promoting action and the expression inducing effect on the glucose transporter gene (GLUT1) in human cardiomyocytes (CMC) of BA-SUP obtained using "a buffer comprising only salts and low-concentration glucose" and BA prepared from feeder-free human ESC.
[Fig. 18] Figures showing mitochondrial protein/gene expression within fine particles (ultracentrifugal sedimentation sample) (2,000 G = cell debris, 10,000 G = microvesicles, 160,000 G = exosome fraction, SUP = culture supernatant) present in BA-SUP obtained using "a buffer comprising only salts and low-concentration glucose" and BA prepared from feeder-free human ESC using a "minimum required medium".
[Fig. 19] figures showing a wound-healing assay using BA-SUP obtained using BA prepared from feeder human ESC using a "minimum required medium".

### [Description of Embodiments]

Hereinafter, embodiments of the present invention will be explained based on the Examples and the like shown in the drawings. The embodiments are not limited to the following examples, and the design can be appropriately changed by using conventionally known techniques such as those mentioned in the above documents without departing from the intents of the present invention.

The present invention provides a composition comprising a supernatant of brown adipocytes. The supernatant and the composition have an action of improving glucose metabolism. In the present invention, the term "comprising a supernatant" means that a supernatant component is comprised, and it maybe a composition comprising a supernatant from which water and salt have been removed. That is, the composition comprising the supernatant of the present invention includes a composition comprising the supernatant or a desalted purified product thereof. The composition comprises at least one, preferably two or more, or three or more components derived from brown adipocytes and having a glucose metabolism-improving action, and may further comprise a pharmaceutically acceptable carrier or vehicle.

The supernatant of brown adipocytes of the present invention can be obtained from desired brown adipocytes, and for example, brown adipocytes derived from pluripotent stem cells can be used. In the present invention, the pluripotent stem cell may be any cell having pluripotency capable of inducing differentiation of brown adipocytes. Typical examples include ES-like cells such as ES cells and iPS cells. These cells may be cells that express alkaline phosphatase, which is an index of ES-like cells. Here, the ES-like cell refers to a pluripotent stem cell having properties and/or morphology similar to those of an ES cell. In addition, pluripotent stem cells can be passaged for a long period of time, which can be confirmed by the fact that the proliferative property is not lost even after passaging, for example, every 3 days for 15 times or more, preferably 20 times or more, 25 times or more, 30 times or more, 35 times or more, or 40 times or more. Pluripotent stem cells also preferably express endogenous OCT3/4 or Nanog, more preferably both. Furthermore, pluripotent stem cells preferably express TERT and exhibit telomerase activity (activity to synthesize telomeric repeat sequences). In addition, pluripotent stem cells preferably have the ability to differentiate into mesoderm, and preferably have the ability to differentiate into three germ layers (endoderm, mesoderm, ectoderm) (which can be confirmed, e.g., in teratoma and/or embryoid body formation). More preferably, pluripotent stem cells generate germline chimeras by transplanting into blastocysts. Pluripotent stem cells capable of germline transmission are called germline-competent pluripotent stem cells. Confirmation of these phenotypes can be performed by well-known methods (WO2007/69666; Ichisaka T et al., Nature 448 (7151): 313-7, 2007).

Specifically, for example, pluripotent stem cells encompass cells with pluripotency capable of inducing differentiation into brown adipocytes, such as embryonic stem cells (ES cells), induced pluripotent stem cells (iPS cells), testis stem cells, adult stem cells, and Muse cells.

As ES cells, desired ES cells capable of inducing differentiation into brown adipocytes can be used, and examples include KhES-1, KhES-2, and KhES-3 (Suemori et al., Biochem Biophys Res Commun 345: 926-932, 2006; Cellosaurus ID CVCL_B233; Cellosaurus ID CVCL_B231, CVCL_B232, CVCL_B233, respectively; RIKEN cell bank IDs HES0001, HES0002, HES0003), more preferably, KhES-1 and KhES-3, and most preferably KhES-3 but are not limited to these. As the ES cells, those that have already been prepared, those that have been prepared without embryo destruction, and the like can also be used.

The method of obtaining and establishing pluripotent stem cells is known. For example, in the case of ES cells, with permission from the Ministry of Education, Culture, Sports, Science and Technology, they can be obtained from a domestic establishing institution (Kyoto University or National Center for Child Health and Development) or obtained or purchased from an overseas facility (private companies, universities, etc.). In addition, iPS cells may also be produced by a desired method known to those skilled in the art. For example, in the case of a method using a Sendai virus (SeV) vector, they can be established by culturing commercially available human fibroblasts or the like in a medium to which a Sendai virus vector carrying a reprogramming factor expression unit is added. An example of a Sendai virus vector carrying a reprogramming factor expression unit includes CytoTune^{™}-iPS (ID Pharma Co., Ltd.). Human iPS cells prepared using a retroviral vector can be purchased from the RIKEN BioResource Center or received from Kyoto University or the National Center for Child Health and Development.

The iPS cells are not particularly limited, and desired iPS cells capable of inducing differentiation into brown adipocytes can be used. The origin is not particularly limited, and can be from fibroblasts, vascular endothelial cells, hepatocytes, and such, but those that do not have a foreign gene inserted into the genome are preferred.

Brown adipocytes (BA) are one type of adipocytes, and methods for identifying them morphologically, physiologically, and/or by expression of marker genes are already known. Typically, brown adipocytes may have multilocular lipid droplets. By multilocular lipid droplets is meant a plurality of small spherical lipid droplets existing in the cytoplasm, and their existence is confirmed by observation using an optical microscope or electron microscope. As a guide, the number of lipid droplets is, for example, 5 or more, preferably 10 or more, more preferably 15 or more, detected in a photograph of a cell section in a cross section including a nucleus when observed with an electron microscope.

Further, the brown adipocytes of the present invention may have in the vicinity of the multilocular lipid droplets a string-like mitochondria which are fused long vertically comprising a plurality of ladder-shaped cristae. By the string-like mitochondria which are fused long vertically comprising a plurality of ladder-shaped cristae is meant mitochondria that have fused with each other in the long axis direction and have become long, and while exhibiting a string-like morphology, inside the mitochondria, disc-shaped cristae that extend vertically with respect to the long axis direction from one end to the other throughout the entire circumference of the inner membrane are densely arranged parallel to each other.

Further, the brown adipocytes of the present invention may express a brown adipocyte marker genes. Known brown adipocyte markers include, for example, UCP1, PRDM16, PGC1α, Cyt-c, CIDE-A, ELOVL3, PPARα, EVA1, and NTRK3. The brown adipocytes of the present invention express, any one or more, two or more, three or more, four or more, five or more, or all of the adipocyte marker genes selected from the group consisting of UCP1, PRDM16, PGC1α, Cyt-c, CIDE-A, ELOVL3, PPARα, EVA1, and NTRK3. Preferably, the brown adipocytes of the present invention are UCP1 and/or PRDM16 positive, and more preferably also PPARγ positive. The induction of expression of the marker gene can be confirmed by RT-PCR or the like.

Further, the brown adipocytes of the present invention have the function of burning fat. For example, brown adipocytes store lipid components as multilocular lipid droplets in the cytoplasm and thereby retain them in a condition where the total surface area is large. In the vicinity of these lipid droplets, a large number of mitochondria, which are highly active in oxidative phosphorylation by the electron transport chain, are present in the form of long fusions in the long axis direction and with ladder-shaped cristae developed inside. Furthermore, the brown adipocytes produced by the present invention express a group of genes for converting the energy obtained by oxidative phosphorylation into heat energy.

Therefore, the brown adipocytes of the present invention exert the effect of actively taking in the lipid components in the environment and burning/eliminating them.

In addition, brown adipocytes may have the action where the expression of genes involved in mitochondrial amplification and heat production such as PRDM16 and UCP1 is increased upon addition of isoproterenol, which is an adrenergic β receptor agonist.

The function of brown adipocytes can be confirmed by increase in cell temperature or oxygen consumption when the adrenergic β receptor agonist isoproterenol and such is added, or by increase in temperature of a transplanted site when brown adipocytes are transplanted into mice or such, and the adrenergic β receptor agonist isoproterenol and such is administered to those mice.

### [General preparation of brown adipocytes]

In the present invention, the origin of brown adipocytes is not particularly limited, and they may be obtained by using a desired isolation or production method. For example, brown adipocytes produced from pluripotent stem cells can be used. The method for producing brown adipocytes from pluripotent stem cells is not particularly limited, and for example, can be produced by a desired *in vitro* production method or the like. For example, when pluripotent stem cell-derived brown adipocytes are produced using cytokines, cell aggregates are formed from pluripotent stem cells by a method including step (A) below, and brown adipocytes are differentiated from the cell aggregates by a method including step (B):
(A) Step of producing cell aggregates by conducting a non-adhesive culture of pluripotent stem cells in the presence of hematopoietic cytokines in a serum-free environment.
(B) Step of adhesively or non-adhesively culturing cell aggregates in the presence of hematopoietic cytokines to prepare brown adipocytes.

As a basal component of the medium used in the above-mentioned production of brown adipocytes, a general-purpose basal medium can be appropriately used. By a serum-free environment is meant culturing without adding serum such as bovine fetal serum to the medium. In the above method, it is not necessary to add serum in every step, and the method can be carried out without adding serum at all in any step. For example, a general-purpose basal medium includes, but is not limited to, IMDM, IMDM/F12, DMEM, DMEM/F12, RPMI, and the like. In addition, examples of serum-free culture medium include S-Clone SF-B (EIDIA Co., Ltd.), S-Clone SF-03 (EIDIA Co., Ltd.), ASF-104 (AJINOMOTO CO., Inc.), ASF-104N (AJINOMOTO CO., Inc.), X-VIVO 10 (Lonza group Ltd.), X-VIVO 15 (Lonza group Ltd.), and such. Preferred basal media include IMDM, IMDM/F12, DMEM, DMEM/F12, RPMI, or a mixture thereof, and in particular, a mixture of IMDM and Ham's F12, such as a 1:1 mixture of IMDM and Ham's F12 (IMDM/F12) can be preferably used.

Further, vitamins (for example, ascorbic acid) and/or amino acids (for example, L-glutamine) and such may be suitably added to the medium for culturing, but may also be not added. In addition, protein components such as insulin, transferrin, albumin, and serum substitutes (GIBCO^{™} PFHM-II Protein-Free Hybridoma Medium, Life Technologies, Inc., etc.) may be added to the medium as appropriate for culturing, but may also be not added. In particular, protein components such as albumin and serum substitutes such as PFHM-II need not be added. If added, these may be added at the concentration of, for example, 5 mg/ml bovine serum albumin (BSA), 1% volume synthetic lipid solution (e.g. Life Technologies, Inc.), 1% volume x 100 insulin-transferrin-selenium (ITS-A) (e.g. Life Technologies, Inc.), 450 µM α-monothioglycerol (MTG) (e.g. Sigma-Aldrich, Inc.), 2 mM L-Glutamine (e.g. Life Technologies, Inc.), 5% volume GIBCO^{™} PFHM-II Protein-Free Hybridoma Medium (PFHII) (e.g., Life Technologies, Inc.), and 50 µg/ml Ascorbic acid. Any one or more of these may be added, or all may be added.

Here, examples of hematopoietic cytokines used in step (A) include BMP4, VEGF, SCF, Flt3L, IL6, and IGF2, and BMP4 is particularly preferable. One type of cytokine such as BMP4 may be used alone, but three or more types of cytokines may also be mixed and used. Furthermore, all 6 types of cytokines may be mixed and used.

The concentration to be used may be determined as appropriate. Examples include BMP4 (1-50 ng/ml, preferably 10-30 ng/ml), VEGF (0.5-20 ng/ml, preferably 1-10 ng/ml), SCF (1-50 ng/ml, preferably 10-30 ng/ml), Flt3L (0.5-20 ng/ml, preferably 1-5 ng/ml), IL6 (0.5-20 ng/ml, preferably 1-5 ng/ml), and IGF2 (0.5-20 ng/ml, preferably 1-10 ng/ml), but is not limited thereto.

A specific example of a hematopoietic cytokine cocktail includes BMP4 (20 ng/ml), VEGF (5 ng/ml), SCF (20 ng/ml), Flt3L (2.5 ng/ml), IL6 (2.5 ng/ml), and IGF2 (5 ng/ml).

An example of a non-adhesive culture in step (A) includes culturing in a culture apparatus (for example, at 37 °C in a 5% CO₂ incubator) while maintaining the floating state in a culture medium without letting the cells adhere to the bottom surface of a culture dish, using a general-purpose low-adsorption culture dish and a semi-solid medium, a hanging drop culture method, a rotary culture using a bioreactor, or the like, but it is not limited thereto. The low adsorption culture vessel used for suspension culture may be any vessel in which pluripotent stem cells do not adhere to the bottom surface thereof. Examples include 2-methacryloxyethyl phosphorylcholine (MPC)-coated culture dish (Thermo Fisher Scientific Inc.), Hydro cell^{™} (Cell Seed Inc.), and the like.

For example, in step (A), pluripotent stem cells are placed in a general-purpose low-adsorption culture vessel or the like using the above-mentioned medium for producing cell aggregates, and cultured at 37 °C for 8-10 days, for example, in a 5% CO₂ incubator, while maintaining the suspended state in the medium without allowing adhesion to the bottom surface of the vessel.

During that time, for example, half of the medium may be replaced with a fresh one every 3 days. When exchanging the medium, for example, tilt the low-adsorption culture vessel about 30 degrees and allow it to stand for about 1 minute to confirm that the cell aggregates are completely submerged, and then gently suck out half of the culture supernatant only with a pipette. After that, add the same amount of fresh medium for producing cell aggregates and uniformly disperse the cell aggregates while gently shaking the entire low-adsorption culture vessel.

The separation and removal of feeder cells from the pluripotent stem cells is preferably done as follows: for example, a suspension of pluripotent stem cells recovered by a detaching solution treatment is allowed to stand in a centrifuge tube for about 30 seconds to selectively precipitate only the pluripotent stem cells, the cells are resuspended using a medium for producing cell aggregates, and then this is suspension-cultured in a low-adsorption culture vessel.

Cell aggregates are formed by step (A). By cell aggregates is meant spherical (or indefinite shape) cell crops formed by culturing pluripotent stem cells or the like in a suspended state in a medium, which may have various sizes and tissue structures, and which may be connected to each other.

Many of the pluripotent stem cell-derived cell aggregates have a spherical or similar morphology with a filled interior, but sometimes they have an indefinite shape or may be fused with each other. In rare cases, the inside may be hollow. The size varies from those that can be confirmed with an optical microscope (diameter 100 to 300 µm) to those that can be easily confirmed with the naked eye (a diameter of 300 to 1000 µm).

The cells of the cell aggregate generated in step (A) are cultured in step (B). The medium used in step (B) is similar to that used in step (A), and the same applies to the basal medium and additives. The culture can be carried out, for example, in a serum-free environment. Examples of hematopoietic cytokines used in step (B) include BMP7, VEGF, SCF, Flt3L, IL6, and IGF2, and BMP7 is particularly preferable. One type of cytokine such as BMP7 may be used alone, or three or more types of cytokines may be mixed and used, or all six types of cytokines may be mixed and used.

The concentration to be used may be appropriately determined. Examples of hematopoietic cytokines include, but are not limited to, BMP7 (1-50 ng/ml, preferably 5-20 ng/ml), VEGF (0.5-20 ng/ml, preferably 1-10 ng/ml), SCF (1-50 ng/ml, preferably 10-30 ng/ml), Flt3L (0.5-20 ng/ml, preferably 1-5 ng/ml), IL6 (0.5-20 ng/ml, preferably 1-5 ng/ml), and IGF2 (0.5-20 ng/ml, preferably 1-10 ng/ml).

A specific example of a hematopoietic cytokine cocktail includes BMP7 (10 ng/ml), VEGF (5 ng/ml), SCF (20 ng/ml), Flt3L (2.5 ng/ml), IL6 (2.5 ng/ml), and IGF2 (5ng/ml).

Step (B) may be an adhesive culture or a non-adhesive culture. The cell culture vessel may be any vessel generally used for cell culture, and a general-purpose cell culture vessel may be used. An example of an adhesive or non-adhesive culture in step (B) includes seeding directly onto or to a coated general-purpose cell culture vessel, and culturing. When a non-contact culture is carried out in step (B), culturing can be carried out following step (A), in which case it is not always necessary to collect the cells after step (A). In that case, step (A) and step (B) are combined into one step. When transferring to another culture vessel, the cells of the aggregate generated in step (A) may be collected and transferred to the other vessel.

The culture conditions for inducing differentiation can be appropriately set depending on the type of pluripotent stem cells used. For example, the cells are cultured in a 5% CO₂ incubator at 37 °C for about a week.

When performing an adhesive culture, a coating treatment may be applied in order to increase cell adhesion. For the coating treatment, for example, an aqueous protein solution such as 0.1% porcine gelatin is placed in a culture vessel and left at room temperature for about 10 minutes. In addition, a general-purpose culture vessel having increased cell adhesion (Corning^{™} CellBIND^{™} Surface, Corning Inc., etc.) may be used. It is preferable to replace the medium with a fresh one roughly every three days during the culture.

Typically, to obtain pluripotent stem cell-derived brown adipocytes from pluripotent stem cells, pluripotent stem cells are separated from feeder cells such as mouse embryonic fibroblasts (MEFs) and then using a BMP4-added medium for preparing cell aggregates, the cells are cultured for about 8 to 10 days in a general-purpose, low-adsorption culture vessel, while maintaining a floating state. The cell aggregates produced thereby are then cultured in a BMP7-added brown adipocyte differentiation medium, in a general-purpose cell culture vessel or in a vessel coated with a protein component such as gelatin. As a result, the production of pluripotent stem cell-derived brown adipocytes from pluripotent stem cells is achieved in about 10 to 15 days.

The pluripotent stem cells, which are the starting material, refer to stem cells having pluripotency, and examples include ES cells, iPS cells, testis stem cells, adult stem cells, and Muse cells. The pluripotent stem cells may be human pluripotent stem cells. In this case, pluripotent stem cells are a group of cells derived from humans and retaining pluripotent differentiation potential. This includes human ES cells, human iPS cells, human testis stem cells, human adult stem cells, human Muse cells, and the like.

### [Preparation of a supernatant of brown adipocytes]

In the present invention, a supernatant of brown adipocytes is prepared from pluripotent stem cell-derived brown adipocytes and the like produced as described above. The supernatant may be prepared by using a suitable known method, but by using a salt buffer, the present invention developed a method of obtaining a supernatant that does not comprise nutritional components such as amino acids and additives such as cytokines contained in culture media. This method includes the following steps:
(1) replacing the culture media of brown adipocytes with a salt buffer,
(2) culturing the brown adipocytes in the salt buffer, and
(3) recovering the salt buffer.

Since the salt buffer does not comprise amino acids, vitamins, proteins, etc., it is possible to obtain a brown adipocyte supernatant that does not comprise exogenous contaminants. In particular, the brown adipocyte supernatant thus obtained is preferable because it does not comprise exogenous cytokines.

The salt buffer is not particularly limited, and a desired salt buffer capable of suspending cells can be used. Specifically, for example, a desired Krebs-Ringer solution, for example, Krebs-Ringer-Hepes (KRH), Krebs-Ringer-Tris (KRT), Krebs-Ringer Bicarbonate, Krebs-Ringer-Hepes-Bicarbonate (KRHB), Krebs-Ringer-Tris Bicarbonate (KRTB) and such can be given.

Furthermore, the salt buffer preferably comprises a sugar such as glucose (salt-glucose buffer). In the present invention, it was found that a supernatant of brown adipocytes having a significant metabolism-improving ability can be obtained not only when a salt-glucose buffer comprising a high concentration of glucose is used, but also when using a salt-glucose buffer comprising a low concentration of glucose. Here, a high concentration glucose means, for example, 10 mM or more, preferably 11 mM or more, 12 mM or more, 13 mM or more, 14 mM or more, 15 mM or more, or 16 mM or more. The high-concentration glucose may also be, for example, 16.8 mM or less. In addition, a low-concentration glucose means, for example, less than 10 mM, preferably less than 9 mM, less than 8 mM, less than 7 mM, less than 6 mM, less than 5 mM, less than 4 mM, or less than 3 mM. The low-concentration glucose may also be, for example, 2.8 mM or more. The salt-glucose buffer of the present invention is preferably a salt-glucose buffer comprising a low-concentration of glucose, and specifically, a salt-glucose buffer comprising about 2.8 mM glucose. Here, "about" means, for example, within the range of ± 10%, preferably within the range of ± 7%, ± 5%, or ± 3%.

The time for culturing with a salt-glucose buffer or the like in step (2) may be appropriately determined. Due to the lack of nutritional components in the salt-glucose buffer, the viability of brown adipocytes may decrease after a long-term culture, and the metabolism-improving action of the supernatant may decrease. On the other hand, it is not possible to prepare a supernatant comprising a sufficient concentration of soluble factors by culturing for a short time. Therefore, the basic composition (including glucose concentration) of the salt-glucose buffer and the optimal culture time for recovering a supernatant having the metabolism-improving action are determined. For example, the culture time of step (2) may be 3 hours or more, 4 hours or more, 5 hours or more, 8 hours or more, 10 hours or more, 12 hours or more, 15 hours or more, 16 hours or more, 20 hours or more, 24 hours or more, 36 hours or more, 48 hours or more, 60 hours or more, 72 hours or more, 84 hours or more, 96 hours or more, 100 hours or more, 108 hours or more, or 120 hours or more, and for example, may be within 10 days, within 9 days, within 8 days, within 7 days, within 6 days, or within 5 days. For example, it can be 5 to 72 hours, 6 to 60 hours, 7 to 48 hours, 8 to 36 hours, 9 to 24 hours, 12 to 24 hours, 13 to 20 hours, or about 16 hours, but is not limited to these.

The step of collecting the supernatant of brown adipocytes in the present invention may be any means capable of separating the cell component and the supernatant component from the culture, regardless of whether it is a suspension culture or an adhesive culture, and spontaneous precipitation, centrifugal sedimentation, filtering, and such can be given as examples.

In a preferred embodiment, the supernatant of brown adipocytes of the present invention is a supernatant that has been separated from brown adipocytes. For example, the supernatant of brown adipocytes of the present invention is a supernatant that does not comprise live cells, and more preferably a supernatant that does not comprise cells (including live cells and dead cells).

Further, the supernatant of brown adipocytes of the present invention may comprise organelles such as mitochondria, or may be that in which organelles are isolated or concentrated (precipitation fraction) or removed (supernatant fraction) by ultracentrifugation or the like. The brown adipocyte supernatant of the present invention comprising mitochondria has a high respiratory ability, an active oxygen-eliminating action, and a wound healing-promoting action. In addition, mitochondria can be efficiently obtained from the brown adipocytes of the present invention or the supernatant thereof. A known method can be appropriately used for separating mitochondria, and for example, as described above, the mitochondria can be separated by ultracentrifugation or the like. The acceleration in centrifugal treatment may be suitably adjusted, and for example, it may be 50 KG or less, 30 KG or less, 20 KG or less, 10 KG or less, 7 KG or less, 6 KG or less, 5 KG or less, 4 KG or less, 3 KG or less, or 2 KG or less. For example, it can be suitably adjusted within the range of 0.1 to 30 KG, 0.2 to 20 KG, or 0.3 to 10 KG, but is not limited to this.

### [Feeder-free pluripotent stem cell culture system]

The present invention also provides brown adipocytes produced from pluripotent stem cells maintained and cultured in a feeder-free system. By preparing brown adipocytes from pluripotent stem cells of a feeder-free system, there is no concern of contamination by feeder cells or components derived from feeder cells, enabling the preparation of purer brown adipocytes and their supernatants. The brown adipocytes and the supernatant thereof thus prepared are also suitable for clinical application.

The feeder-free culture system for maintaining undifferentiated pluripotent stem cells in the present invention is not limited as long as the pluripotent stem cells can be passage-cultured for proliferation while maintaining them in an undifferentiated state. Examples of medium include Essential 8 Medium (Thermo Fisher Scientific) and StemFit^{™} (Ajinomoto Healthy Supply Co., Inc.), and examples of coating agents for culture dishes include Gibco^{™} Vitronectin Human Protein, Natural (Thermo Fisher Scientific) and iMatrix-511 (Nippi, Inc.), etc.

While not being limited thereto, a specific example of the procedure for maintaining and culturing pluripotent stem cells without a feeder is as follows ("Transition of StemFit^{™} AK02N on-feeder iPS cells to feeder-free" (iPS portal, Inc.; ips-guide.com/wp-content/themes/ips-guide/pdf/products/ak02n/AK02N_protocol04.pdf); Takahashi K, et al., Cell, 2007 Nov.30; 131 (5): 861-72; Nakagawa M, et al. Scientific Reports 4: 3594 (2014); Kyoto University Center for iPS Research and Application (CiRA) HP: "Feeder-free establishment and maintenance culture of human iPS cells" (www.cira.kyoto-u.ac.jp/j/research/images/protocol/pdf/hipsprotocolFf_140311.pdf)).

### I. Preparation of medium

To a required amount StemFit^{™} AK02N (Ajinomoto Co., Inc.), add 10 mM Y-27632 (Rho kinase inhibitor) at 1/1000^{th} of the volume of the culture medium, and mix well (final concentration 10 µM) (hereinafter, referred to as AK02N+Y).

### II. 6-well plate coating

1. Add 1.5 mL each of Laminin-511 E8 solution (iMatrix-511) diluted to 3.2 µg/mL with PBS (-) into the wells to be coated (coating volume is 4.8 µg/well (0.5 µg/cm²)). At that time, first put the required amount of PBS (-) into a 50 mL tube or such, add Laminin-511 E8 thereto so as to make 3.2 µg/mL, mix well immediately, and swiftly dispense 1.5 mL to each well.
2. Allow to react for 60 min or more in a 5% CO₂ incubator at 37 °C, take out from the incubator after the reaction, add 0.75 mL/well of StemFit^{™} AK02N, mix well, and then discard the supernatant. Then, add 1.5 mL/well of AK02N+Y medium and place in the incubator for 15 min or more (up to 60 min).

### III. Passaging

1. After confirming cells with a phase-contrast microscope, remove the medium, add 1 mL of PBS (-) to wash, and discard the PBS (-). Add 600 µL/well of CTK solution (prepared by adding to 30 mL of distilled water, 2.5% trypsin 5.0 mL, 1.0 mg/mL collagenase IV 5.0 mL, 0.1 M CaCl₂ 0.5 mL, and 10 mL of KSR) and mix well. Allow to react at room temperature for 1-2 minutes and detach feeder cells from the plate. After washing by adding 1 mL of PBS (-), discard the PBS, add 0.5X TrypLE^{™} Select at 300 µL/well, mix well, and allow to react in a 5% CO₂ incubator at 37 °C (after confirming that the permeability of center of the colony has sufficiently increased, move on to the next task (check that the cell-cell adhesion is broken and each cell has become rounded). Then, remove 0.5X TrypLE^{™} Select (if the cells are detached at this point, proceed to 2).

Wash with 2 mL/well PBS (-), remove the PBS (-), add 1 mL/well of StemFit^{™} AK02N+Y, and detach the cells with a cell scraper. Check if the cells have detached using a phase-contrast microscope, pipette about 10 times, and collect cells to a new tube (add medium to make the total volume 1.5 mL. The total volume can be changed as appropriate). After that, do a cell count and seed 13,000 live cells into one well of a 6-well plate coated with Laminin (the cell adhesion is very strong so swirl the plate immediately after seeding to evenly spread the cells). Incubate in a 37 °C, 5% CO₂ incubator, and on the next day, replace with StemFit^{™} AK02N that does not contain Y-27632.

### 2. (Continuation for the case where the cells are detached in the midst of the procedure)

Add 700 µL of StemFit^{™} AK02N (1000 µL in total) and pipette about 10 times to separate the cells. Centrifuge at 800 rpm (160 x g) at 22 °C for 5 min, remove the supernatant, break the pellet by tapping, add 1 mL/well of StemFit^{™} AK02N, pipette 6 times to separate the cells, and do a cell count. Then, seed 13,000 live cells into one well of a 6-well plate coated with Laminin (the cell adhesion is very strong so swirl the plate immediately after seeding to evenly spread the cells) (seeding volume is adjusted according to the clone used). Incubate in a 37 °C, 5% CO₂ incubator, and on the next day, replace with StemFit^{™} AK02N that does not contain Y-27632.

It is desirable to change the medium one day after the day of passaging, and after that, every other day, or every two days when the cell density is low, and every day when the cell density is high. Cell passage is done in around 8 days ± 1 day.

The present invention's method for producing brown adipocytes from pluripotent stem cells cultured feeder-free includes the following steps:
(1) maintaining and culturing pluripotent stem cells feeder-free,
(2) non-adhesively culturing the cells of step (1) in a serum-free environment to form cell aggregates, and
(3) culturing the cells of step (2) in a serum-free environment to differentiate into brown adipocytes.

Further, the present invention's method for producing a brown adipocyte supernatant from pluripotent stem cells cultured feeder-free includes the following step in addition to the above steps:
(4) incubating the brown adipocytes of step (3) in a salt buffer and collecting the supernatant thereof.

That is, the present invention's method for producing a brown adipocyte supernatant from pluripotent stem cells cultured feeder-free includes the following steps:
(1) maintaining and culturing pluripotent stem cells feeder-free,
(2) non-adhesively culturing the cells of step (1) in a serum-free environment to form cell aggregates,
(3) culturing the cells of step (2) in a serum-free environment to differentiate into brown adipocytes, and
(4) incubating the brown adipocytes of step (3) in a salt buffer and collecting the supernatant thereof.

As described above, the salt buffer is not particularly limited, and a desired salt buffer capable of suspending cells such as KRH, KRT, KRHB, and KRTB can be used. In addition, the salt buffer preferably comprises a sugar such as glucose. The glucose concentration is as described above. In addition, the culture time with the buffer in step (4) is also as described above.

In the above method for producing a supernatant of brown adipocytes from pluripotent stem cells cultured feeder-free, in steps (2) (cell aggregate-forming step) and (3) (brown adipocyte differentiation step), a cytokine cocktail may be added similarly to steps (A) and (B) described above, respectively. In that case, examples of cytokines added in step (2) include cytokines such as BMP4, VEGF, SCF, Flt3L, IL6, and IGF2 as in step (A), and BMP4 is particularly preferable. One type of cytokine such as BMP4 may be used alone, or three or more types of cytokines may be mixed and used. Furthermore, all six types of cytokines may be mixed and used. Examples of cytokines added in step (3) include BMP7, VEGF, SCF, Flt3L, IL6, and IGF2 as in step (B), and BMP7 is particularly preferable. One type of cytokine such as BMP7 may be used alone, or three or more types of cytokines may be mixed and used, or all six types of cytokines may be mixed and used. Particularly preferable combinations of cytokines and the concentration of cytokines are the same as those mentioned for steps (A) and (B).

In step (2), the cells of step (1) can be dispersed to single cells or a similar level, and aggregates can be formed from those cells. Here, similar level to single cells means, for example, that the average number of cells contained in a particle consisting of cells or cell clusters contained in a cell suspension is a few cells or less, preferably 2 to 3 cells or less, 2 cells or less, or less than 2 cells, and for example, it means that in a cell suspension, the proportion of single cells is 20% or more, 30% or more, 40% or more, 50% or more, 60% or more, 70% or more, 80% or more, or 90% or more.

The culture period can be suitably adjusted, and in step (2), cell aggregates may be generated by culturing for, for example, 8 to 10 days. As in the above step (A), the medium may be replaced with a new medium on a regular basis. Further, similarly to above step (B), step (3) may be an adhesive culture or a non-adhesive culture, and the period can be appropriately adjusted, and for example, the culture may be performed for about one week.

Similar to step (B) above, step (3) may be a contact culture or a non-contact culture . When performing a non-contact culture, the culture can be performed following step (2), in which case it is not always necessary to collect the cells after step (2). In that case, step (1) and step (2) are combined into one step. When transferring to another culture vessel, the cells of the aggregates generated in step (2) may be collected and transferred to the other vessel.

As described below, when pluripotent stem cells cultured feeder-free are used, brown adipocytes can be generated without adding cytokines, which are essential for the case using feeder-cultured pluripotent stem cells.

### [Manufacturing of brown adipocytes without cytokine cocktails]

For example, the method of the present invention for producing brown adipocytes from pluripotent stem cells cultured feeder-free includes the following steps:
(1) maintaining and culturing pluripotent stem cells feeder-free,
(2) dispersing the cells of step (1) and non-adhesively culturing in a serum-free environment without adding a cytokine cocktail comprising BMP4, VEGF, SCF, Flt3L, IL6, and IGF2,
(3) culturing the cells of step (2) in a serum-free environment without adding a cytokine cocktail comprising BMP7, VEGF, SCF, Flt3L, IL6, and IGF2.

### [Step 1]

First, in step (1), pluripotent stem cells cultured feeder-free are prepared. Maintaining and culturing pluripotent stem cells feeder-free can be appropriately carried out as described above.

### [Step 2]

Next, the pluripotent stem cells of step (1) are dispersed, and a non-adhesive culture is carried out in a serum-free environment without adding a cytokine cocktail comprising BMP4, VEGF, SCF, Flt3L, IL6, and IGF2 to form cell aggregates.

In the present invention, the production of pluripotent stem cell-derived cell aggregates can be done by any process as long as pluripotent stem cells can be detached from the culture dish, dispersed to single cells or a similar level, and then reaggregated, and maybe a process used for forming cell aggregates such as culture in a low adsorption culture dish, hanging drop culture method, or rotation culture in a bioreactor.

When cell aggregates are formed using pluripotent stem cells cultured using a feeder, a cytokine cocktail comprising BMP4, VEGF, SCF, Flt3L, IL6, and IGF2 is added, but when using pluripotent stem cells cultured feeder-free it is not necessary to add a cytokine cocktail comprising BMP4, VEGF, SCF, Flt3L, IL6, and IGF2. Therefore, in step (2) above, no cytokine cocktail comprising BMP4, VEGF, SCF, Flt3L, IL6, and IGF2 is added (i.e., the cells are not cultured in the presence of exogenous BMP4, VEGF, SCF, Flt3L, IL6, and IGF2).

Some of the above cytokines may be added in order to maintain a high cell viability and increase the efficiency of cell aggregate formation, but in a preferred embodiment, in step (2), 2 or more, more preferably 3 or more, 4 or more, 5 or more, more preferably all six cytokines selected from the group consisting of BMP4, VEGF, SCF, Flt3L, IL6, and IGF2 are not added. In particular, it is possible to avoid addition of cachexia-causing IL6 and/or VEGF with angiogenic potential.

Namely, a minimum required medium to which neither serum nor cytokine is added can be used as the medium of step (2). In the present invention, basal medium constituting the "minimum required medium" used for inducing the differentiation of pluripotent stem cells into brown adipocytes may be any medium in which pluripotent stem cell-derived cell aggregates are formed and brown adipocytes are produced. The medium used is the same as described above, and a general-purpose basal medium can be appropriately used. Examples of general-purpose basal media include IMDM, IMDM/F12, DMEM, DMEM/F12, RPMI, and the like, but are not limited thereto. Preferred basal media include IMDM, IMDM/F12, DMEM, DMEM/F12, RPMI, or mixtures thereof, and in particular, a mixture of IMDM and Ham's F12, such as a 1:1 mixture of IMDM and Ham's F12 (IMDM/F12) can be preferably used. Further, in the present method, it is not necessary to add serum in all the steps, and it is possible to carry out the method without adding serum at all in any step.

To the medium, it is possible to add bovine serum albumin (BSA), α-monothioglycerol (α-MTG), and/or protein free hybridoma mix (PFHMII, Gibco #12040-077, Life Technologies, Inc.), which are said to have the effect of increasing cell viability, but cell aggregates are formed even in a minimum required medium without these additions, and the cells of the formed cell aggregates have the ability to differentiate into brown adipocytes. Therefore, it is possible to produce brown adipocytes using a minimum required medium without using the animal-derived protein BSA, or PFHM-II, which comprises a physiologically active molecule. Insulin, transferrin, and/or serine may be added to the medium. For example, insulin and/or transferrin can be added. In addition, ITS-A (Insulin-Transferrin-Selenium-Sodium Pyruvate) may be added to the medium. The amounts added can be appropriately adjusted.

The culture period can be suitably adjusted, and cell aggregates may be generated by culturing for 8 to 10 days, for example. As in the above step (A), the medium may be replaced with a new medium on a regular basis.

### [Step 3]

Next, the cells of the cell aggregates formed in step (2) are cultured in a serum-free environment. The culture in this step does not have to be an adhesive culture, and may be an adhesive or non-adhesive culture. In the case of a non-contact culture, the culture can be performed following step (2), and in that case, as it is not necessary to collect the cells after step (2), steps (2) and (3) can be carried out as a single step, and brown adipocytes can be prepared from pluripotent stem cells in one step. When transferring to another culture vessel in step (3), the cells of the aggregates generated in step (2) may be collected and transferred to the other vessel.

That is, the present invention relates to a method of producing brown adipocytes from pluripotent stem cells in one step, comprising the step of dispersing pluripotent stem cells maintained and cultured feeder-free and non-adhesively culturing them in a serum-free environment. A step of performing an adhesive culture may or may not be included after this step. If a step of performing an adhesive culture is not included, the non-adhesive culture may simply be continued. In addition, neither BMP4, VEGF, SCF, Flt3L, IL6, and IGF2-comprising cytokine cocktails nor BMP7, VEGF, SCF, Flt3L, IL6, and IGF2-comprising cytokine cocktails are added in any of these steps. Preferably, none of the BMP4, BMP7, VEGF, SCF, Flt3L, IL6, and IGF2 cytokines are added. In a more preferred embodiment, no cytokine is added in any of these steps. That is, the present invention provides a method for producing brown adipocytes from pluripotent stem cells in one step without adding cytokines (that is, without using exogenous cytokines) using a feeder-free culture system.

When brown adipocytes are prepared from pluripotent stem cells in one step, the serum-free medium used is not particularly limited, but for example, it is preferable to use a serum-free medium to which a lipid is added. In addition, the size of the cell aggregates produced may be appropriately adjusted in order to promote the differentiation into brown adipocytes. For example, the preferred size of the aggregate may be 1500 µm or less, for example 1200 µm or less, 1000 µm or less, 900 µm or less, 800 µm or less, 700 µm or less, 600 µm or less, 500 µm or less, 400 µm or less, 300 µm or less, or 200 µm or less. The size of the aggregates can be controlled by adjusting the size of holes and depressions in the culture equipment.

As the cell culture vessel, those generally used for cell culture can be appropriately used. In the case of adhesive culture, a coating treatment may be applied in order to increase cell adhesion. The coating treatment can be carried out using, for example, an aqueous protein solution such as 0.1% porcine gelatin as described above. In addition, a general-purpose culture vessel having increased cell adhesion (Corning^{™} CellBIND^{™} Surface, Corning Inc., etc.) may be used.

When performing a non-adhesive culture, cells can be cultured using a general-purpose low-adsorption culture dish or semi-solid medium without adhering the cells to the bottom surface of the culture dish and keeping the floating state in the medium. The low-adsorption culture vessel used for suspension culture may be any vessel in which cells do not adhere to the bottom surface thereof, and examples include culturing in a low-adsorption culture dish, a hanging drop culture method, and rotary culture in a bioreactor.

When forming cell aggregates using pluripotent stem cells cultured with a feeder, a cytokine cocktail comprising BMP7, VEGF, SCF, Flt3L, IL6, and IGF2 is added in this step, but the present step which uses pluripotent stem cells cultured feeder-free, it is not necessary to add a cytokine cocktail comprising BMP7, VEGF, SCF, Flt3L, IL6, and IGF2. Thus, no cytokine cocktail comprising BMP7, VEGF, SCF, Flt3L, IL6, and IGF2 is added in present step (3) (i.e., the cells are not cultured with exogenous BMP7, VEGF, SCF, Flt3L, IL6, and IGF2).

In order to maintain a high cell viability and increase the efficiency of brown adipocyte production, some of the above cytokines may be added, but in a preferred embodiment, in present step (3), two or more, more preferably three or more, four or more, five or more, more preferably all six cytokines selected from the group consisting of BMP7, VEGF, SCF, Flt3L, IL6, and IGF2 are not added. In particular, it is possible not to add cachexia-causing IL6 and/or VEGF with angiogenic potential.

When no cytokine is added in any of steps (2) and (3), or when the cytokines added in steps (2) and (3) are the same, a medium having exactly the same composition (minimum required medium) can be used in steps (2) and (3). In this case, steps (2) and (3) can be carried out as one step (step (2')). In this case, the method of the present invention for producing brown adipocytes from pluripotent stem cells cultured feeder-free includes the following steps:
(1) maintaining and culturing pluripotent stem cells feeder-free,
(2') dispersing the cells of step (1), and conducting a non-adhesive culture in a serum-free environment without adding a cytokine cocktail comprising BMP4, VEGF, SCF, Flt3L, IL6, and IGF2 or a cytokine cocktail comprising BMP7, VEGF, SCF, Flt3L, IL6, and IGF2.

The above step (2') may be, for example, a step of dispersing the cells of step (1) and performing a non-adhesive culture in a serum-free environment without adding any cytokine.

By the above series of steps, brown adipocytes can be generated from pluripotent stem cells. As described above, the proportion of brown adipocytes can be measured by staining lipid droplets, measuring the expression of a marker gene, etc.

Since brown adipocytes generated from feeder-free system-derived pluripotent stem cells produced in this manner are not in contact with heterologous cells such as feeder cells or exogenous cytokines during the generation process, they are extremely high-quality brown adipocytes that are not contaminated with or influenced by heterologous cells or exogenous cytokines. By preparing a supernatant using these brown adipocytes, it is possible to obtain a high-quality brown adipocyte supernatant that also does not comprise any factors derived from heterologous cells or exogenous contaminants.

### [Step 4]

The method of obtaining a supernatant of brown adipocytes using a salt buffer includes the following step:
(4) incubating the brown adipocytes of step (3) (or the above step (2')) in a salt buffer and collecting the supernatant thereof.

As described above, the salt buffer is not particularly limited, and a desired salt buffer capable of suspending cells such as KRH, KRT, KRHB, and KRTB can be used. In addition, the salt buffer preferably comprises a sugar such as glucose. The concentration of glucose may be high or low, but preferably a low-concentration of glucose is contained. Specific concentrations are as described above, and for example, a salt buffer comprising 0.5-5 mM, preferably 0.5-5 mM, 0.8-4 mM, 0.9-3.5 mM, 1-3.2 mM, 1.5-3.0 mM, 2-3 mM, for example, about 2.8 mM glucose can be preferably used.

The culturing time with the buffer is also as described above. For example, the culturing time in the above step (4) may be 3 hours or more, 4 hours or more, 5 hours or more, 8 hours or more, 10 hours or more, 12 hours or more, 15 hours or more, 16 hours or more, 20 hours or more, 24 hours or more, 36 hours or more, 48 hours or more, 60 hours or more, 72 hours or more, 84 hours or more, 96 hours or more, 100 hours or more, 108 hours or more, or 120 hours or more, for example, within 10 days, within 9 days, within 8 days, within 7 days, within 6 days, or within 5 days. For example, it can be 5 to 72 hours, 6 to 60 hours, 7 to 48 hours, 8 to 36 hours, 9 to 24 hours, 12 to 24 hours, 13 to 20 hours, or about 16 hours, but is not limited to these.

The step of collecting the supernatant of brown adipocytes in the present invention may be any means as long as it can separate the cell component and the supernatant component from the culture, regardless of whether step (3) is a suspension culture or adhesion culture, and examples include spontaneous precipitation, centrifugal sedimentation, filtering, etc. The same applies to the above step (2').

Further, the culture device used for the series of brown adipocyte cultures may be used under any conditions, and there are no restrictions on the culture device type, temperature, humidity, oxygen concentration, carbon dioxide concentration, etc.

The supernatant of brown adipocytes may be appropriately subjected to treatments such as desalting, purification, and concentration.

The supernatant of brown adipocytes in the present invention may be stored in any manner after preparation, and there are no restrictions on storage methods such as refrigeration, freezing, and freeze-drying.

Further, any solute or any solvent may be added to the supernatant of brown adipocytes in the present invention after preparation. For example, a frozen dry product can be used by dissolving in a KRTB buffer, but it is not limited thereto.

Further, the supernatant of brown adipocytes in the present invention may be chemically modified as appropriate. Such modifications may be made for the purpose of, for example, labeling, addition of tags, improvement of stability, increase of activity, etc., and a specific example includes biotinylation. The activity of the supernatant of brown adipocytes of the present invention is maintained even after modification (Example 16). For example, in the case of chemical modification, a desired functional group of the supernatant component can be modified, and specifically, an amino group can be modified.

The brown adipocytes thus obtained, the supernatant thereof, and the purified product thereof have the excellent property of being substantially free of contaminants derived from heterologous animal cells. The brown adipocytes thus obtained, the supernatant thereof and the purified product thereof can be used as a basic research tool for studying the active factors contained therein and also as a therapeutic tool for obesity and hyperlipidemia.

For example, the brown adipocytes of the present invention or supernatants thereof have an insulin secretion-promoting action on pancreatic β cells, a glucose uptake-promoting action and a glucose transporter gene expression-enhancing action on pancreatic β cells and muscle cells, an *in vivo* blood glucose-reducing action, an insulin secretion-promoting action, an insulin sensitivity-enhancing action, and the like. In addition to lowering blood glucose levels, especially *in vivo,* administration of the supernatant can also reduce the HOMA-IR value, which is an insulin resistance index, thereby increasing insulin sensitivity. As described above, since the brown adipocytes of the present invention, the supernatant thereof, and the desalted purified product thereof have an action of improving glucose metabolism, they can be used for the treatment and prevention of various pathological conditions and symptoms requiring improvement of glucose metabolism.

The brown adipocytes of the present invention or the supernatant thereof are also useful for various cosmetic purposes aimed at reducing or preventing increase in body weight or body fat, slimming (including partial slimming), improving body shape, and the like.

Described is an internal medical treatment or a cosmetic method for the prevention or treatment of obesity, which comprises administering the above-mentioned brown adipocytes, a supernatant thereof, or a purified product thereof.

Similarly, the present disclosure provides an internal medical treatment for improving insulin resistance and/or preventing or treating diabetes or prediabetes, which comprises administering the above-mentioned brown adipocytes, a supernatant thereof, or a purified product thereof.

The internal medical treatments encompass those used in combination with any one of prevention or treatment of obesity, improvement of insulin resistance, prevention or treatment of diabetes, prevention or treatment of hyperlipidemia, treatment for promoting hematopoiesis, and surgeries for creating coronary artery bypass circulation, which comprise administering the above-mentioned brown adipocytes, a supernatant thereof, or a purified product thereof.

The present disclosure also provides preventive and/or therapeutic compositions for various diseases presenting metabolic abnormalities such as obesity and/or overweightness, metabolic syndrome, type 2 diabetes, and type 1 diabetes (particularly slowly progressive type 1 diabetes), comprising the brown adipocytes of the present invention, a supernatant thereof, or a purified product thereof as active ingredient. The present disclosure also provides a cosmetic composition for the purpose of reducing or preventing an increase in body weight or body fat, slimming (including partial slimming), improving body shape, etc., comprising the brown adipocytes of the present invention, a supernatant thereof, or a purified product thereof as an active ingredient.

The present disclosure also provides a pharmaceutical composition for various diseases showing a mitochondrial dysfunction, which comprises the brown adipocytes of the present invention, a supernatant thereof, or a purified product thereof, as an active ingredient. Specifically, the present disclosure provides a preventive and/or therapeutic composition for various diseases presenting a mitochondrial dysfunction such as mitochondrial disease and neonatal heart disease, which comprises the brown adipocytes of the present invention, the supernatant thereof, or a purified product thereof, as an active ingredient.

The present disclosure also provides a pharmaceutical composition for various diseases presenting skin damage, which comprises the brown adipocytes of the present invention, a supernatant thereof, or a purified product thereof, as an active ingredient. Specifically, the present disclosure provides a preventive and/or therapeutic composition for various diseases that present a skin damage such as subcutaneous bleeding, pigmentation due to subcutaneous bleeding, and subcutaneous telangiectasia, which comprises the brown adipocytes of the present invention, the supernatant thereof, or a purified product thereof, as an active ingredient.

The composition comprising the brown adipocytes of the present invention, the supernatant thereof, and/or the purified product thereof may appropriately comprise a pharmaceutically acceptable carrier or vehicle. The composition of the present invention is used as a pharmaceutical composition, for example, a pharmaceutical composition used for improving glucose metabolism, and is also useful as a cosmetic composition. The carrier and vehicle are not particularly limited, and examples include water (for example, sterile water), physiological saline (for example, phosphate buffered saline), glycol, ethanol, glycerol, lactose, sucrose, calcium phosphate, gelatin, dextran, agar, pectin, oils such as olive oil, peanut oil, sesame oil, etc., as well as emulsifiers, suspending agents, surfactants, buffers, flavoring agents, diluents, preservatives, stabilizers, excipients, vehicles, preservatives, and so on.

For example, the brown adipocytes of the present invention can be suspended in physiological saline, a culture medium, a salt buffer, or the like to form a composition. Further, the cell composition may be embedded or attached to a substrate/base material such as a gel, jelly, or solid. Such substrates/base materials include hydrogel, gelatin, collagen, hyaluronic acid, chondroitin sulfate, dermatan sulfate, keratin sulfate, heparin, heparin sulfate, galactosamino-glycuronoglycan-sulfate, glycosaminoglycan, mucopolysaccharide, agarose, agar, or derivatives thereof, and may comprise biomolecules such as osteonectin, fibrinogen, or osteocalcin. When combined with a solid base material, its shape is not particularly limited, and beads, porous materials, sponges, nets, threads or hollow fibers, or sheet-like materials can be used.

For example, cells can be embedded in a fibrin gel to prepare a composition for transplantation or injection into a living body. A fibrin gel can be prepared, for example, by adding thrombin to an aqueous solution of fibrinogen. For example, type I collagen or aprotinin may be added to the aqueous fibrinogen solution. Type I collagen can be suitably neutralized and used.

Further, a composition comprising a supernatant of the present invention or a purified product thereof can be appropriately formulated as an aqueous solution, a lyophilized product, or the like.

Formulation as an injection can be prescribed using a carrier such as distilled water for injection. Examples of an aqueous solution for injection include physiological saline and an aqueous solution comprising other components such as glucose, D-sorbitol, D-mannose, D-mannitol, and sodium chloride. It may also comprise alcohol, propylene glycol, polyethylene glycol, a nonionic surfactant, and the like.

The pharmaceutical composition and cosmetic composition are preferably administered by parenteral administration. For example, they can be formulated as agents for an injection, a nasal administration, a pulmonary administration, a transdermal administration, and the like. The administration may be done systemically or locally, for example, through intravenous injection, intramuscular injection, intraperitoneal injection, subcutaneous injection, or the like. The details of the administration method can be appropriately selected depending on the age and symptoms of the patient. The dose can be set, for example, in the range of 0.0001 mg to 1000 mg/kg body weight at a time, converted to a dry product of the supernatant. Alternatively, for example, a dose of 0.001 to 100,000 mg per patient can be set, but it is not limited to this. When cells are administered, for example, 1 x 10² to 1 x 10⁸ cells can be administered per site, and the administration site may be or one to several sites, a dozen sites, or dozens of sites, but is not limited thereto. A person skilled in the art can appropriately determine an appropriate dose and administration method considering the weight, age, symptoms, and such of the patient.

The cells and supernatants can be administered to a desired subject. The subject of administration can be appropriately selected, and includes, for example, mammals, specifically rodents such as mice, rats, and guinea pigs, non-rodent animals such as cows, pigs, goats, and rabbits, desired primates, for example, non-human primates such as monkeys, and also humans.

### [Example]

Hereinafter, the present invention will be described in more detail with reference to Examples, but it is not limited thereto. In addition, all publications and other references cited herein are incorporated as part of the present specification.

[Example 1] The blood glucose-reducing action when a human ESC-derived brown adipocyte supernatant prepared with "a buffer comprising only salts and a high-concentration of glucose" was subcutaneously administered to mice:
Differentiation into brown adipocytes (BA) was induced by the method described in [Reference Example 4] using a human ESC KhES-3 strain (Suemori et al., Biochem Biophys Res Commun 345: 926-932, 2006) maintained and cultured on mouse fetal fibroblasts (MEF). The differentiation medium was removed from mature BA on the 10th day of induction of differentiation (2nd day of adhesion culture in a culture dish with a radius of 6 cm after floating for 8 days), 2 ml of Krebs-Ringer-HEPES (KRH) buffer comprising 16.8 mM glucose (NaCl: 128 mM, KCl: 5 mM, CaCl₂ 2.7 mM, MgSO₄ 1.2 mM, Na₂HPO₄: 1 mM, HEPES (pH 7.4): 20 mM, Glucose: 16.8 mM) was added thereto, this was cultured for 16 hours in a carbon dioxide incubator (at 37 °C, 5% CO₂), and the supernatant was collected (BA-SUP). This was injected subcutaneously into six 10-week-old ICR strain mice (12.5 µl/g body weight). The mice were fasted thereafter, and 16 hours later, blood was collected from the tail vein, and the fasting blood glucose level, insulin level, and triglyceride (TG) level were measured. After this, 1g/kg of glucose solution was orally administered using a feeding tube, and the blood insulin level was measured 15 minutes later. As a control, an equal amount converted in terms of body weight (12.5 µl/g body weight) of 16.8 mM glucose-added KRH buffer was administered to six mice, and the same experiment was performed.

As a result, the fasting blood glucose level of the BA-SUP-administered mouse group was significantly lower than that of the control mouse group (Fig. 1A, left). The HOMA-IR value (fasting insulin level (µU/ml) x fasting blood glucose level (mg/dl)/405), which is an insulin resistance index, also decreased significantly (Fig. 1A, middle). Thus, it was shown that insulin sensitivity was enhanced by the administration of BA-SUP. On the other hand, there was no difference in fasting TG levels between the two groups (Fig. 1A, right). Subcutaneous transplantation of human ESC (KhES-3 strain)-derived BA into mice resulted in a decrease in both fasting blood glucose level and fasting TG level (NPL 19). This suggests that, among the metabolism improving actions exhibited by BA, the effect of improving glucose metabolism is the action of soluble factor BATokine(s) secreted by BA, and the effect of improving lipid metabolism is due to BA taking in neutral fat from the blood and burning it.

Thus, it is thought that BA exerts an action of improving glucose metabolism through BATokine(s) independently of body heat production due to fat burning. Since the blood insulin level 15 minutes after the oral glucose loading was increased in the BA-SUP-administered mice (Fig. 1B), this suggests that BA-SUP not only enhances insulin sensitivity, but also promotes insulin secretion.

[Example 2] The glucose transporter gene Glut4/GLUT4 expression-inducing action of human ESC-derived BA supernatant prepared with "a BA differentiation medium" when added to skeletal muscle cells:
Using human ESC (KhES-3 strain) maintained and cultured on mouse fetal fibroblasts (MEF), BA was prepared by the method described in [Reference Example 4]. Then, the differentiation medium was removed from mature BA on Day 10 and a fresh differentiation medium was added (medium exchange). After culturing in a carbon dioxide incubator (37 °C, 5% CO2) for 16 hours, the supernatant was recovered (BA-SUP). As a control, a fresh differentiation medium was cultured in the carbon dioxide incubator (37 °C, 5% CO2) using a gelatin-coated dish, and the supernatant recovered after 16 hours was used as a control supernatant (Control SUP).

Using mouse myoblast cell line C2C12 obtained from the American Type Culture Collection (ATCC), myotube cells were prepared on a 96-well plate according to the method recommended by ATCC, then the medium was removed, BA-SUP or Control SUP was added, and the cells were cultured in a carbon dioxide incubator (37 °C, 5% CO₂) for 16 hours. Then, the cells were collected, and the expression of Glut4, which is a glucose transporter gene that functions in skeletal muscle cells, and of Actin, beta (ACTB), which is a control gene for correction, was measured by a quantitative RT-PCR method.

As a result, the expression level of Glut4 was significantly increased by the addition of BA-SUP (Fig. 2A, left). In addition, similar results were obtained when the Glut4 expression level was measured using Glyceraldehyde-3-phosphate dehydrogenase (GAPDH) as a correction control gene in an independently conducted experiment (Fig. 2A, right).

After adding BA-SUP prepared by the method described in the previous paragraph to human skeletal muscle cells (SkMC, Takara Bio Co., Ltd.) and culturing in a carbon dioxide incubator (37 °C, 5% CO2) for 16 hours, the cells were collected and the expression of the GLUT4 gene and the GAPDH gene as a correction control gene was examined.

As a result, the expression of GLUT4 was significantly increased by the addition of BA-SUP (Fig. 2B).

[Example 3] The insulin secretion-promoting action on pancreatic beta cells of a supernatant of human ESC/iPSC-derived BA prepared with "a buffer comprising only salts and a high-concentration of glucose":
SUP of human ESC-derived BA was prepared in the same manner as in [Example 1]. That is, BA was induced to differentiate from human ESC maintained and cultured on MEF (NPL 19), the differentiation medium was removed from the mature BA on Day 10, and then a KRH buffer comprising 16.8 mM glucose was added. The supernatant was collected after culturing in a carbon dioxide incubator (37 °C, 5% CO2) for 16 hours. Meanwhile, MIN6 cells, which is a mouse pancreatic beta cell line (distributed by Jun-ichi Miyazaki, adjunct professor at the Office for University-Industry Collaboration, Osaka University (at present)), was cultured in a 96-well plate using the recommended method (Miyazaki et al., Endocrinology 127: 126-132, 1990). After washing with PBS buffer, this was cultured for one hour in 2.8 mM glucose-comprising KRH buffer (low glucose treatment), the cell supernatant was removed, and then BA-SUP diluted to 1/10^{th} with control SUP (16.8 mM glucose-comprising KRH buffer) or the control SUP was added, and cultured for two hours in a carbon dioxide incubator (37 °C, 5% CO₂). Then, the supernatant of MING cells was collected, and the insulin concentration was measured by the ELISA method (ultra-sensitive mouse insulin measurement kit, Morinaga Institute of Biological Science, Inc.).

As a result, the amount of insulin secreted in the BA-SUP-added group was significantly increased as compared to the control SUP-added group (Fig. 3A).

Similar results were obtained in a study conducted using BAs prepared from human iPSC (strain prepared from human umbilical vein endothelial cells HUVEC, PTL 1, NPL 19) (Fig. 3B).

[Example 4] The insulin secretion-promoting action on pancreatic beta cells of a supernatant of human ESC-derived BA prepared with "a buffer comprising only salts and a low-concentration of glucose":
A human ESC-derived BA-SUP having a low glucose concentration was prepared by the same procedure as in [Example 1]. That is, BA was prepared from the KhES-3 strain maintained and cultured on MEF, the differentiation medium was removed from mature BA on Day 10, and then the Krebs-Ringer-TRIS-Bicarbonate (KRTB) buffer comprising 2.8 mM glucose was added (NaCl: 119 mM, KCl: 4.74 mM, CaCl₂: 1.19 mM, MgCl₂ 1.19 mM, KH₂PO₄: 1.19 mM, NaHCO₃: 25 mM, TRIS (pH 7.4): 10 mM, Glucose: 2.8 mM) and cultured in a carbon dioxide incubator (37 °C, 5% CO₂) for 16 hours after which the supernatant (BA-SUP) was collected. As a control, a 2.8 mM glucose-comprising KRHB buffer was used, and the effect on insulin secretion was evaluated using MIN6 cells by the same procedure as in [Example 3].

As a result, the amount of insulin secreted in the BA-SUP-added group was significantly increased as compared to the control SUP-added group (Fig. 4).

[Example 5] Induction of BA differentiation from "human pluripotent stem cells maintained in a feeder-free culture system":
Human ESC (KhES-3 strain) under maintenance culture using Essential 8 Flex Medium Kit (Thermo Fisher Scientific, USA) in culture dishes coated with 30-fold diluted Corning^{™} Matrigel^{™} Matrix (Corning, USA) were collected using a detachment solution (0.25% Trypsin, 1 mg/ml, Type IV Collagenase; 1 ml, 20% Knockout Serum Replacement (KSR)^{™}; 0.01 mM CaCl₂/PBS), and the cells were dispersed into single cells using TripLE^{™} Express Enzyme (Thermo Fisher Scientific) in the presence of RevitaCell^{™} Supplement (x100) (Thermo Fisher Scientific), a ROCK inhibitor. These cells were then seeded into PrimeSurface^{™} 96V well culture plates (Sumitomo Bakelite Co., Ltd.) at 2 × 10⁴ cells/well to 6 × 10⁴ cells/well, and spheres were prepared by a culture using the medium for producing cell aggregates described in [Reference Example 4] (Fig. 5A). Spheres were collected on Day 8 and cultured in the brown adipocyte-inducing medium described in [Reference Example 4] using collagen I-coated culture dishes (AGC Techno Glass Co., Ltd.). On Day 10, phase-contrast microscopy observed multilocular lipid droplets characteristic of mature BA (Fig. 5B left), which were stainable with the lipid stain Oil Red O (Fig. 5B middle) and the expression of the UCP1 protein involved in the heat-production ability of BA was confirmed by immunostaining (Fig. 5B, right). In addition, when cells were collected over time during the differentiation process and quantitative RT-PCR was performed, MYF, a dorsal extensor group myoblast marker as a BA precursor, was found to be induced peaking on Day 6 (myoblast differentiation stage, NPL 19). It was also confirmed that the expression of transcription factor EBF2 and the transcriptional regulator PRDM16, which are required for the commitment of myoblasts (having a two-directional ability to differentiate into BA and skeletal muscle cells) to differentiate into BA, was induced in the latter half of differentiation, and the expression of transcription factor PPARγ required for adipocyte maturation (lipid droplet formation) was induced in the late differentiation stage (Fig. 5C).

[Example 6] Insulin secretion-promoting action of a human BA supernatant prepared with "a buffer comprising only salts and a low-concentration of glucose" using BA produced from "human pluripotent stem cells maintained in a feeder-free culture system":
Human ESC (KhES-3 strain) being maintained and cultured using culture dishes coated with StemFit^{™} AK02N (Ajinomoto Healthy Supply Co., Inc.) and Vitronectin (Vitronectin (VTN-N) Recombinant Human Protein, Truncated, A14700, Thermo Fisher Scientific) was used to induce differentiation into BA with the same procedure as in [Example 5]. Using the prepared mature BA, a SUP was prepared with "a buffer comprising only salts and a low-concentration of glucose" with the same procedure as in [Example 4] (BA-SUP). Using this BA-SUP, the effect on insulin secretion was investigated using MIN6 cells in the same manner as in [Example 4].

As a result, it was found that BA-SUP (Fig. 6, black column) exerts an insulin secretion-promoting ability comparable to high-concentration insulin stimulation (Fig. 6, gray column) (Fig. 6).

[Example 7] Induction of BA differentiation using "a medium comprising no cytokine cocktail" from "human pluripotent stem cells maintained in a feeder-free culture system":
Using human ESC (KhES-3 strain) under maintenance culture in culture dishes coated with StemFit^{™} AK02N (Ajinomoto Healthy Supply Co., Inc.) and Vitronectin (Id., Thermo Fisher Scientific), cells were detached, collected, and dispersed into single cells by the same procedure as in [Example 5], and this was seeded into 96-well-type EZSPHERE^{™} (AGC Techno Glass Co., Ltd.) at 1 × 10⁵ cells/well to produce cell aggregates and induce differentiation into BA. At this time, differentiation was induced using a medium prepared by removing the cytokine cocktail from the medium used for producing cell aggregates described in [Reference Example 4] and the medium for inducing brown adipocytes. Then it was examined whether signals of the autocrine/paracrine factor group secreted by the human ESCs themselves can induce a highly-oriented, spontaneous BA differentiation in spheres prepared from human ESC dispersed into single cells.

As a result, cell aggregates were formed from the KhES-3 strain even when the medium for producing aggregates and the medium for inducing brown adipocytes not comprising cytokine cocktails were used, similar to the conventional method of preparing cell aggregates, and also, the gene expression of markers PRDM16, PPARγ, and CEBPB was sufficiently induced (Fig. 7). Similarly, when differentiation was induced from human iPSC (derived from BJ cells) using a medium from which a cytokine cocktail has been removed, cell aggregates were formed. Expression of the PRDM16 gene, a BA marker, in cells on Day 8 of differentiation was 155-fold higher than that of undifferentiated iPSCs.

[Example 8] Insulin secretion-promoting action of a human BA supernatant prepared with "a buffer comprising only salts and a low-concentration of glucose" using BA induced from "human pluripotent stem cells maintained in a feeder-free culture system" using "a medium comprising no cytokine cocktail":
hESC (KhES-3 strain) BA was prepared in the same manner as in [Example 7], and BA-SUP was prepared using Krebs-Ringer-Tris-Bicarbonate (KRTB) buffer (NaCl: 119 mM, KCl: 4.74 mM, CaCl₂ 1.19 mM, MgCl₂ 1.19 mM, KH₂PO₄: 1.19 mM, NaHCO₃: 25 mM, Tris (pH 7.4):10 mM, Glucose 2.8 mM), which is "a buffer comprising only salts and a low-concentration of glucose", in the same manner as in [Example 6]. Using this, in the same manner as in [Example 4], the amount of insulin in the supernatant was measured 2 hours after addition to MIN6 cells, using Infinite^{™} M1000 PRO (Tecan Japan Co., Ltd.) by the New HTRF^{™} High Range Insulin Assay. As a negative control, a low glucose (2.8 mM glucose)-comprising KRTB buffer and a high glucose (16.8 mM glucose)-comprising KRTB buffer were used.

As a result, the supernatant (BA-SUP) prepared from "brown adipocytes derived from human pluripotent stem cells maintained in a feeder-free culture system in a medium comprising no cytokine cocktail" using "a buffer comprising only salts and a low-concentration of glucose" also showed insulin secretion-promoting activity (Fig. 8).

[Example 9] Induction of BA differentiation using "a minimum required medium comprising no cytokine cocktail" from "human pluripotent stem cells maintained in a feeder-free culture system":
Among the various components contained in a differentiation medium, regarding bovine serum albumin (BSA), α- monothioglycerol (α-MTG), and protein free hybridoma mix (PFHMII, Gibco # 12040-077, Life Technologies, Inc.), which are added to replace the effect of serum in a general-purpose "serum-free medium" and are said to have the effect of ensuring cell viability, it was investigated whether they are essential for inducing brown adipocyte differentiation, and whether cells that differentiated into cell lines other than brown adipocytes could be eliminated by removing them. That is, in the same manner as in [Example 7], BA differentiation was induced from human ESCs (KhES-3, KhES-1 strains) maintained in a feeder-free culture system via a single-cell dispersion operation, and the status of BA differentiation induction was compared using the "medium comprising no cytokine cocktail" described in [Example 7] and the medium from which BSA, α-MTG, and PFHMII were further removed therefrom.

As a result, for the KhES-3 strain, even if BA differentiation was induced using a medium from which all of these components had been removed (minimum required medium), cell aggregates were formed in the same manner as when a cytokine-comprising medium was used (Fig. 9A), and the expression of BA markers PRDM16 and PPARγ was not inferior compared to when the cytokine-comprising medium was used (Fig. 9B). In addition, on the 10th day of differentiation induction, abundant mitochondria (Fig. 9C, green) and multilocular lipid droplets (Fig. 9C, red), which are cell morphologies characteristic of mature BA, were observed. When the expression of genes encoding the cytokines (IL6, VEGF, BMP4, Flt-3L, SCF) added to the differentiation-inducing medium in the method of [Reference Example 4] was measured by a quantitative RT-PCR method, it was found that the expression of these genes was induced during the differentiation process (Fig. 9D). From this result, it can be envisaged that when differentiation was induced using the "minimum required medium", the expression of endogenous genes encoding the cytokines contained in the cytokine cocktail was induced, and they functioned in an autocrine/paracrine manner, inducing BA differentiation even under the condition where no exogenous cytokines were added.

Next, for the KhES-1 strain also, BA differentiation was induced using the minimum required medium. As a result, the expression of the BA markers PRDM16 and PPARγ was induced at a level comparable to that using the cytokine-comprising medium (Fig. 9E), and on the 10th day of differentiation induction, abundant mitochondria (Fig. 9F, green) and multilocular lipid droplets (Fig. 9F, red), which are cell morphologies characteristic of mature BA, were observed.

As described above, in both of the two human ESC strains (KhES-3 strain and khES-1 strain) tested, a BA differentiation induction equivalent to when using a cytokine cocktail-comprising medium as used by prior art was achievable even by using a minimum required medium comprising no cytokines.

The composition of the minimum required medium used in this section is as follows. 1:1 mixed medium of IMDM (I3390, Sigma Chemical) and Ham's F12 (087-08335, FUJIFILM Wako Pure Chemical Corporation), 1:100 Chemically Defined Lipid Concentrate (Gibco # 11905-031, Thermo Fisher Scientific), 1:100 insulin-transferrin-selenium (ITS-A, Thermo Fisher Scientific), 2 mM Glutamax II (L-alanine-L-glutamine dipeptide; Gibco # 35050-061, Thermo Fisher Scientific), and 50 µg/ml ascorbic acid-2-phosphate (A-8960, Sigma Chemical)

[Example 10] Insulin secretion-promoting action of a human BA supernatant prepared with "a buffer comprising only salts and a low concentration of glucose" using BA produced from "feeder-free human ESC" in a "minimum required medium":
In the same manner as in [Example 9], BA differentiation was induced from human ESC (KhES-3 strain) maintained in a feeder-free culture system in a minimum required medium, and in the same manner as in [Example 8], BA-SUP was prepared with "a buffer comprising only salts and a low concentration of glucose". The BA-SUP thus prepared was added to MIN6 cells, and the amount of insulin secreted was measured by the New HTRF^{™} High Range Insulin Assay method similarly to [Example 8].

As a result, BA-SUP prepared by this method also showed insulin secretion-promoting activity (Fig. 10).

[Example 11] Glucose uptake-promoting action on pancreatic beta cells of a human BA supernatant prepared with "a buffer comprising only salts and a low concentration of glucose" using BA produced in a "minimum required medium" from "feeder-free human ESC":
In the same manner as in [Example 10], BA prepared from human ESC (KhES-3 strain) maintained in a feeder-free culture system in a minimum required medium was used to prepare BA-SUP using "a buffer comprising only salts and a low concentration of glucose". This was added to MIN6 cells, and the effect on glucose uptake was analyzed using the Glucose Uptake-Glo^{™} Assay (Promega). Specifically, 1.2 × 10⁵ MIN6 cells were seeded into a 96-well plate, cultured for 72 hours, then cultured in a low glucose-comprising KRTB buffer (glucose 2.8 mM) for 2 hours. Then the medium was removed, BA-SUP or control SUP was added, washed 3 times with PBS after 3 hours to wash out glucose, then 50 µl of 1 mM 2-deoxyglucose (2-DG) was added, followed by the addition of stop solution after 75 minutes and then the neutralizing solution, and 60 minutes after the addition of the luminoassay substrate, the luminescence was measured with a luminometer.

As a result, the glucose uptake ability of MIN6 cells was enhanced by the administration of BA-SUP (Fig. 11A).

[Example 12] Insulin secretion-promoting action of a human BA supernatant prepared with "a buffer comprising only salts and a low concentration of glucose" using BA produced in a "minimum required medium" from "feeder-free human ESC" by "1 step" of only a suspension culture:
Using human ESC (KhES-3 strain) maintained and cultured using StemFit^{™} AK02N (Ajinomoto Healthy Supply Co., Inc.) and a culture dish coated with Vitronectin, detaching and collecting, and dispersing into single cells were carried out in the same procedure as in [Example 7], the cells were then floated in a minimum required medium at a density of 3 × 10⁵ cells/ml. This was then rotation cultured using a 30 mL single-use bioreactor (BWV-S03A, Able Corporation.), on a 6-channel magnetic stirrer (BWS-S03N0S-6, Able Corporation) installed inside a carbon dioxide incubator (37 °C, 5% CO₂) at a rate of 55 rotations/min. After culturing for 10 days while exchanging half of the medium (minimum required medium) with a fresh one every two days, it was washed with 10 ml of the "buffer comprising only salts and a low concentration of glucose" described in [Example 8]. Then, the cells were suspended in 7.5 ml of the "buffer comprising only salts and a low concentration of glucose" described in [Example 8], and seeded (1.5 ml/dish) into five Nunclon 60 mm dishes (non-coated). After culturing in a carbon dioxide incubator (37 °C, 5% CO₂) for 16 hours, the cells were centrifuged to prepare the BA-SUP. Using this BA-SUP, the effect on insulin secretion was investigated using MIN6 cells in the same manner as in [Example 4]. Meanwhile, as in [Example 7], cell aggregates prepared in the minimum required medium using EZSPHERE^{™} (AGC Techno Glass Co., Ltd.) were adherent-cultured with collagen I coated dish (AGC Techno Glass Co., Ltd.) to induce BA differentiation. Then, the BA-SUP prepared with the "buffer comprising only salts and a low concentration of glucose" described in [Example 8] was also prepared, and in the same manner as in [Example 4], the effect on insulin secretion was investigated using MIN6 cells.

As a result, no difference was observed in insulin secretion-promoting activity between BA-SUP prepared by suspension culture in all steps using a bioreactor and BA-SUP prepared through two types of culture steps, suspension culture and adhesion culture (Fig. 12).

[Example 13] Effect of ultracentrifugation on insulin secretion-promoting activity of BA-SUP prepared with "a buffer comprising only salts and a low concentration of glucose" from BA obtained from feeder-free human ESC in a "minimum required medium":
In the same manner as in [Example 12], BA differentiation was induced from human ESC (KhES-3 strain) maintained in a feeder-free culture system with a minimum required medium, and BA-SUP was prepared using "a buffer comprising only salts and a low concentration of glucose". In addition, a part of the BA-SUP was subjected to ultracentrifugal treatment at 160,000 G, separated into supernatant components and sediment components (pellets) and collected, and the effect on insulin secretion was investigated using MIN6 cells.

As a result, most of the insulin secretion-promoting activity of BA-SUP was recovered in the supernatant component after the 160,000G ultracentrifugation treatment (Fig. 13). However, insulin secretion-promoting activity was also observed in the pellet component (exosome fraction) after the160,000 G ultracentrifugation treatment, although it was a very small amount (Fig. 13). The above suggests that the action of insulin secretion-promoting activity in the BA-SUP is mainly due to soluble factor BATokine(s) (BATokine(s) in the narrow sense), but exosome BATokine(s) (BATokine(s) in the broad sense) are also present in the BA-SUP in a small amount. This result also suggests that there are several different bioactive factors in the BA-SUP that have a metabolism-improving action.

[Example 14] Evaluation of *in vivo* heat-producing ability of BA prepared from non-feeder human ESC in a "minimum required medium":
Human ESC (KhES-1 strain) and human iPS cells (201B7 strain) maintained in a feeder-free culture system using StemFit^{™} and iMatrix-511 coated dishes were collected and suspended in the minimum required medium. This was seeded on an Elplasia^{™} 3D Discovery tool (Kuraray Co., Ltd.) at a density of 1.8 × 10⁵ cells/well (i.e., 2 × 10³ cells/spheroid). After that, BA differentiation was induced in "one step" without an adhesive culture while exchanging half of the medium with a fresh minimum required medium every 2 days. The cells were collected on Day 11, washed once with PBS, ice-cooled in a pellet-down state, and embedded in 30 µl of fibrin gel. Here, fibrin gel was prepared as follows: to 89.3 µl of an aqueous solution (2.8 mg/ml) of fibrinogen (Fibrinogen from bovine plasma Type I-S, Sigma Aldrich, product number F8630) were added 6.7 µl of a neutralized type I collagen solution (Collagen Type I Rat Tail, Corning, product number 354236) (3.0 mg/ml), and 3 µl of aprotinin solution (aprotinin from bovine lung saline solution, Sigma Aldrich, product number A6279) to make 99 µl of a solution, and thereafter was added 1 µl of a 50 U/ml thrombin solution (Thrombin from bovine plasma lyophilized powder, Sigma Aldrich, product number T4648). The neutralized type I collagen solution is a mixture of 5.55 µl collagen solution and 1.15 µl neutralizing solution (0.67 µl 10XPBS + 0.13 µl 1N NaOH + 0.35 µl distilled water). A "BA-embedded gel" was prepared by adding 30 µl of this fibrin gel solution to an ice-cooled tube comprising the cell pellet and suspending the cells well. As a control, a gel was prepared by introducing 30 µl of fibrin gel solution into a tube without the cell pellet. These gels were transplanted into mice according to the method described in NPL 19 to evaluate the heat producing ability *in vivo.* Specifically, "fibrin gel" or "BA-embedded fibrin gel" was injected using a P1000 chip through a 5 mm skin incision into the right buttock of an ICR strain mouse whose buttocks had been depilated in advance (Fig. 14A, 14C, arrows) (Note: Due to the characteristics of fibrin gel, the gel travels from the incision to the subcutaneous tissue and spreads widely to the subcutaneous tissue of the buttocks). 48 hours later, the sympathetic receptor agonist isoproterenol was intraperitoneally administered, and six minutes after the administration, the mice were photographed with an infrared camera centering on the buttocks.

As a result, the skin temperature of the buttocks increased significantly in the mice transplanted with the BA-embedded gel after the administration of isoproterenol compared with the mice transplanted with the gel alone (Figs. 14B and 14D).

Thus, it was confirmed that the BA prepared with the "minimum required medium" is a "functional BA" that exerts heat-producing ability *in vivo.*

[Example 15] Glucose uptake-promoting action on human skeletal muscle cells (SkMC) of a BA-SUP prepared with "a buffer comprising only salts and a low concentration of glucose" using BA produced in "minimum required medium" from feeder-free human ESC:
In the same manner as in [Example 10], BA differentiation was induced from human ESC (KhES-3 strain) maintained in a feeder-free culture system in a cell-required medium, and BA-SUP was prepared using "a buffer comprising only salts and a low concentration of glucose (2.8 mM) (KRTB)". Then, the effect on glucose uptake of human skeletal muscle cells (SkMC) (CC-2561, Lonza) was evaluated using a dedicated kit (Glucose Uptake-Glo^{™} Assay, J1342, Promega). The specific procedure is as follows.

SkMC with a passage number of 4 was seeded into a 24-well plate using SkGM-2^{™} Bullet Kit^{™} (Lonza) (10⁵ cells/well) and cultured at 37 °C in a 5% carbon dioxide incubator. After 8 days, the cells were washed with 0.5 ml of PBS, the medium was replaced with 500 µl of KRTB or BA-SUP, and the cells were cultured for 3 hours. After that, the cells were washed with 0.5 ml of PBS, 0.25 ml of 1 mM 2-deoxyglucose (2-DG, 040-06481, Fuji Film Wako Pure Chemical Industries, Ltd.) dissolved in PBS was added, the cells were cultured for another 10 minutes, and 125 µl of stop solution (included in the kit) was added. Then, the cells were collected and lysed, and the glucose uptake ability was measured according to the instructions. The glucose uptake ability was evaluated by the value (luminescence/protein) obtained by correcting the fluorescence value of luciferin produced by treatment with the cell lysate and the kit with the amount of protein in the cell lysate. The amount of protein was measured by BCA assay (T9300A, Takara Bio, Inc.) In addition, as positive control experiments on glucose uptake-promoting action, Glucose Uptake-Glo^{™} Assay was performed on DMEM medium comprising no glucose (09891-25, Nacalai Tesque Co., Ltd.) and DMEM medium comprising no glucose added with 1mM insulin (I9278, Sigma-Aldrich).

As a result, when SkMC was cultured in BA-SUP, glucose uptake was promoted as compared to when cultured in KRTB, and the degree was similar to the effect of 1 mM insulin (Fig. 15). From the above, it was shown that BA-SUP has an action of promoting glucose uptake of human skeletal muscle cells.

[Example 16] Glucose uptake-promoting action on human skeletal muscle cells (SkMC) of a biotinylated BA-SUP prepared with "a buffer comprising only salts and a low concentration of glucose" using BA produced in a "minimum required medium" from feeder-free human ESC:
In the same manner as in [Example 15], BA differentiation was induced from human ESC (KhES-3 strain) maintained in a feeder-free culture system in a cell-required medium, and BA-SUP was prepared using "a buffer comprising only salts and a low concentration of glucose (2.8 mM) (KRTB)". Then, 1 ml of BA-SUP or 1 ml of KRTB (comprising 2.8 mM glucose) was freeze-dried, dissolved in 900 µl of distilled water, and then applied onto PD MiniTrap G-10 (GE Healthcare Japan Co., Ltd.), which is a desalting column. Then, after eluting with 500 µl PBS, the amount of protein was measured by a BCA assay, and then using the EZ-Link^{™} Sulfo-NHS-LC-Biotinylation Kit (Thermo Fisher Scientific), which is an amino group biotinylation kit, a biotinylation reaction was conducted according to the instruction manual attached. Then, 300 µl was collected from the reaction sample and applied to PD MiniTrap G-10, eluted with 500 µl of DMEM medium comprising no glucose (09891-25, Nacalai Tesque, Inc.), and 200 µl of which was freeze-dried. Thereafter, in the same manner as in Example 15, the effect on glucose uptake of SkMC was evaluated. The amount used in the assay corresponds to 480 µl of BA-SUP, and the conditions are almost the same as those of the experiment shown in Example 15.

As a result, it was confirmed by a blot using streptavidin that many proteins in the BA-SUP were biotinylated (Fig. 16A). In addition, it was confirmed that even after biotinylation, a glucose uptake-promoting action equivalent to that of 1 mM insulin was shown for SkMC (Fig. 16B). From the above, it was found that the glucose uptake-promoting action is maintained even if the proteins present in BA-SUP are biotinylated.

[Example 17] Glucose uptake-promoting action on human cardiomyocytes of BA-SUP prepared with "a buffer comprising only salts and a low concentration of glucose" using BA produced from feeder-free human ESC:
In the same manner as in [Example 6], BA differentiation was induced from human ESC (KhES-3 strain) maintained in a feeder-free culture system in a required medium, and BA-SUP was prepared using "a buffer comprising only salts and a low concentration of glucose (KRTB)". Then, the effect on the glucose uptake of human cardiac myocytes (HCM) was evaluated using a dedicated kit (Glucose Uptake-Glo^{™} Assay, J1342, Promega). The specific procedure is as follows.

HCM (C-12810, Promocell) were seeded into a 12-well plate (5 × 10⁴ cells/well) using the Myocyte Growth Medium Kit (C-22170, Promocell) and cultured at 37 °C in a 5% carbon dioxide incubator. After 7 days, the medium was replaced with 1 ml of DMEM medium comprising no glucose (09891-25, Nacalai Tesque Co., Ltd.), and after culturing at 37 °C for 3.5 hours in a 5% carbon dioxide incubator, it was washed 3 times with KRTB (comprising 2.5 m glucose), and HCM were cultured in a 5% carbon dioxide incubator at 37 °C for 2.5 hours using 1) KRTB (comprising 2.5 m glucose) 1 ml, 2) KRTB (comprising 2.5 m glucose) plus 100 nM Insulin 1 ml, or 3) a mixture of KRTB (comprising 2.5 m glucose) 0.5 ml and BA-SUP 0.5 ml. Then, 10 µl of 1 mM 2-DG was added to each, cultured for 0.5 hours, washed 3 times with 3 ml of PBS, and then the cells were collected to evaluate the glucose uptake capacity of HCM in the same manner as in Example 15.

As a result, it was found that BA-SUP promotes glucose uptake of HCM equivalent to or more than 100 nM insulin (Fig. 17A). In addition, using the recovered HCM, the expression of GLUT1 which is the main glucose transporter in cardiomyocytes was measured by quantitative RT-PCR. As a result, it was found that BA-SUP increased the expression of GLUT1 equivalent to or more than 100 nM insulin (Fig. 17B). From the above, it was considered that BA-SUP promotes glucose uptake by inducing GLUT1 expression in cardiomyocytes.

[Example 18] Recovery of mitochondria-comprising fine particles from BA-SUP prepared with "a buffer comprising only salts and a low concentration of glucose" using BA produced in a "minimum required medium" from feeder-free human ESC:
In the same manner as in [Example 15], BA differentiation was induced from human ESC (KhES-3 strain) maintained in a feeder-free culture system in a cell-required medium, and BA-SUP was prepared using "a buffer comprising only salts and a low concentration of glucose (2.8 mM) (KRTB)". The fine particles contained in the BA-SUP were recovered by a stepwise ultracentrifugal operation, and the presence or absence of mitochondria was examined. The specific procedure is as follows.

BA-SUP was ultracentrifuged with a centrifugal force of 2 KG (G is gravitational acceleration), the supernatant was ultracentrifuged again with 10 KG, and the supernatant of that was ultracentrifuged again with a centrifugal force of 160 KG. The precipitated fractions of each ultracentrifugation treatment were collected, and the presence or absence of mitochondria was evaluated by qPCR using the Human Mitochondrial DNA Monitoring Primer Set (Takara Bio, Inc.).

As a result, the presence of mitochondria was confirmed in the fractions precipitated by ultracentrifugation at 2 KG and 10 KG (Figs. 18A and 18B). Using the precipitated fractions recovered by stepwise ultracentrifugation at 0.3 KG, 2 KG, 10 KG, and 160 KG, Western blotting was performed using exosome markers (CD9, CD81), HSP70 and β-actin known to be secreted extracellularly as fine particles, together with an antibody against the mitochondrial marker protein ATP5A (Fig. 18C). As a result, the presence of mitochondria-derived protein ATP5A was confirmed in the fractions precipitated by ultracentrifugation at 0.3 KG, 2 KG, and 10 KG.

From the above, it was found that mitochondria-comprising fine particles can be efficiently recovered by centrifuging BA-SUP at an acceleration of 10 KG or less and recovering the precipitated fraction.

[Example 19] Evaluation of wound healing ability of BA-SUP by a wound-healing assay, where the BA-SUP is prepared using BA produced in a "minimum required medium" from feeder-free human ESC:
In the same manner as in [Example 15], BA differentiation was induced from human ESC (KhES-3 strain) maintained in a feeder-free culture system using a minimum required medium. On the 8th day of differentiation induction, the culture was transferred to an adhesive culture and replaced with a fresh minimum required medium. After culturing at 37 °C in a 5% carbon dioxide incubator for another 2 days, the culture supernatant was collected on the 10th day of differentiation induction. This was centrifuged at 300 G to remove cell debris, and further ultracentrifuged at 160,000 G to remove the sediment (fine particle fraction), and the supernatant obtained was collected as the BA-SUP. As a negative control, the supernatant obtained by centrifuging the minimum required medium at 300 G and then performing ultracentrifugal treatment at 160,000 G to remove the sediment (fine particle fraction) was used.

Meanwhile, Human Umbilical Vein Endothelial Cells (HUVEC) were cultured in a 12-well plate using a dedicated medium (EGM^{™}-2 Endothelial Cell Growth Medium-2 BulletKit^{™}, Takara Bio Co., Ltd.). Once the cells reached confluence, 1000 µl plastic chips were used to linearly detach cells in the center of the plate (wound formation). After washing with PBS, photographs of the cells were taken with a phase-contrast microscope, and 600 µl each of the minimum required medium or BA-SUP (the fine particle fraction removed from both by ultracentrifugation) was added to the cells. After culturing at 37 °C for 16 hours in a 5% carbon dioxide incubator, photographs of the part where the wounds (damage) were formed were retaken again, and the area of the bottom surface of the plate not covered with cells (damaged part) before and after culturing were compared.

As a result, the detachment part formed by the scraping using plastic chips shrinks after culturing in the minimum required medium or BA-SUP (Fig. 19, left), but the shrinkage rate (wound healing ability) was significantly higher in the BA-SUP-added sample (Fig. 19, right).

Accordingly, it was found that BA-SUP enhances the ability to heal damage of vascular endothelial cells.

### [Reference Example 1] Induction of human induced pluripotent stem cells using Sendai virus vector:

Human umbilical vein endothelial cells (HUVEC) (Lonza group Ltd.) were seeded into a 0.1% porcine gelatin-coated 6-well culture plate at 2.5 × 10⁵ cells/well, and cultured using EGM-2 medium (Lonza group Ltd.) in a 5% CO₂ incubator at 37 °C. After culturing, the cultured cells were infected with the following vectors (a) to (d) having a concentration of MOI = 3 (WO2010/008054).
(a) SeV18 + OCT3/4/TSΔF vector
(b) SeV18 + SOX2/TSΔF vector
(c) SeV18 + KLF4/TSΔF vector
(d) SeV (HNL) c-MYC/TS15ΔF vector

After infection with the vector, on the next day the medium was exchanged with DMEM comprising 10% FBS. Thereafter, the cells were cultured in a 5% CO₂ incubator at 37 °C for 6 days. Next, 9.0 × 10⁵ to 1.5 × 10⁶ of vector-introduced cells detached by Accutase (Innovative Cell Technologies, Inc.) were cultured on 6.0 × 10⁵ (cells) of X-ray-irradiated mouse fetal fibroblasts (MEF) prepared in a gelatin-coated 10 cm culture dish. The next day, the medium was changed from DMEM comprising 10% FBS to a medium for primate ES cells (ReproCELL Inc.) (where FGF2 was added to 5 ng/ml), and cultured in a 3% CO₂ incubator. Medium exchange was performed daily.

Colonies appeared several days after culturing, and human embryonic stem cell-like colonies appeared after culturing for about 20 days. Flat colonies similar to those found in human embryonic stem cells, which are clearly different from pre-induction HUVEC, were observed. The human embryonic stem cell-like colonies were similar in appearance to those previously reported.

These colonies were isolated with a micropipette and then cultured on a new MEF. Stable passaging and growth were possible via a detaching operation using human pluripotent stem cell detaching solution (0.25% trypsin (Life Technologies, Inc.), 1 mg/ml collagenase IV (Wako Pure Chemical Industries, Ltd.) 20% KnockOut^{™} Serum Replacement (Life Technologies, Inc.) 1 mM CaCl₂).

### [Reference example 2] Removal of foreign genes derived from SeV vector:

Cloning was done to obtain a strain in which SeV vector-derived foreign genes were removed from the SeV-iPS cells obtained in Reference Example 1.

Immunostaining with anti-SeV antibody (DNAVEC Corporation) was performed as a guideline for removal of foreign genes derived from SeV vector. SeV-iPS cells were fixed with 10% Mildform (Wako Pure Chemical Industries, Ltd.), and anti-SeV antibody was used as the primary antibody and Alexa Fluor 488-labeled anti-rabbit IgG antibody (Life Technologies, Inc.) was used as the secondary antibody. After staining, observation was done with a fluorescent microscope.

Furthermore, RT-PCR was performed to detect transgenes (OCT3/4, SOX2, KLF4, cMYC) and SeV genome. As a result, it was confirmed that the SeV-iPS cell line obtained in Reference Example 1 was negative for the SeV antigen and did not carry foreign genes derived from the SeV vector.

### [Reference Example 3] Maintenance culture of human embryonic stem cells and human induced pluripotent stem cells:

Human embryonic stem cells (KhES-3) used were those donated by the Institute for Frontier Life and Medical Sciences, Kyoto University. Maintenance cultures of KhES-3 and SeV-iPS cell lines described in Reference Example 1 were done on X-ray radiated MEF using DMEM/F12 (Life Technologies, Inc.) medium comprising 20% Knockout Serum Replacement (KSR) (Life Technologies, Inc.), 5 ng/ml FGF2, 1% non-essential amino acids solution, 100 µM 2-mercaptethanol, and 2 mM L-glutamine.

### [Reference Example 4] Induction of brown adipocytes from human embryonic stem cells and human induced pluripotent stem cells:

To induce differentiation into brown adipocytes, first, in order to separate and remove MEF from KhES-3 and SeV-iPS cell lines, the suspension of pluripotent stem cells recovered by the detaching solution treatment was left to stand in a centrifuge tube for about 30 seconds to selectively precipitate only pluripotent stem cells.

Induction of differentiation into brown adipocytes was performed in the following two steps.

### (1) Step A

The sediment consisting of pluripotent stem cells in Reference Example 2 was floated in 4 ml of a medium for producing cell aggregates (5 mg/ml BSA, 1% volume synthetic lipid solution, 1% volume × 100 ITS-A, 450 µM MTG, 2 mM L glutamine, 5% volume PFHII, and 50 µg/ml ascorbic acid-comprising IMDM/F12 medium including 20 ng/ml BMP4, 5 ng/ml VEGF, 20 ng/ml SCF, 2.5 ng/ml Flt3L, 2.5 ng/ml IL6 , and 5 ng/ml IGF2), this was transferred to a 6-well MPC-coated culture dish, and cultured at 37 °C in a 5% CO₂ incubator. After that, the cells were cultured for 8 to 10 days while changing half of the medium every 3 days. To exchange the medium, the entire MPC-coated culture dish was inclined by about 30 degrees and left to stand for about 1 minute, the cell aggregates were confirmed to be completely submerged, and then half-volume of only the culture supernatant was gently sucked out with a pipette. Then, the same amount of fresh medium for preparing cell aggregates was added, and the cell aggregates were uniformly dispersed while gently shaking the entire MPC-coated culture dish.

### (2) Step B

The pluripotent stem cell-derived cell aggregates prepared above were transferred to a centrifuge tube of about 10 ml and allowed to stand for about 30 seconds to 1 minute to allow the cell components to precipitate, and then the supernatant was removed. 3 ml of a medium for inducing brown adipocytes(5 mg/ml BSA, 1% volume synthetic lipid solution, 1% volume × 100 ITS-A, 450 µM MTG, 2 mM L-Glutamine, 5% volume PFHII, and 50 µg/ml ascorbic acid-comprising IMDM/F12 medium including 10 ng/ml BMP7, 5 ng/ml VEGF, 20 ng/ml SCF, 2.5 ng/ml Flt3L, 2.5 ng/ml IL6, and 5 ng/ml IGF2) was added and centrifuged for 5 minutes at 1100 rpm. This was transferred to a cell culture dish that was reacted beforehand with 0.1% porcine gelatin aqueous solution at room temperature for 10 minutes, and cultured at 37 °C in a 5% CO₂ incubator. After that, the cells were cultured for 1 week while changing the medium every 3 days.

As a result, cells having multilocular lipid droplets (a large number of yolk-like glossy spheres) in the cytoplasm were obtained. The fact that these spheres were lipid droplets was confirmed by Oil Red O staining (a test that stains triglycerides in red).

In addition, through RT-PCR, it was found by electrophoresis that the expression of genes characteristic of brown adipocytes (UCP1, PRDM16, PGC1α, Cyt-c, CIDE-A, ELOVL3, PPARα, EVA1, and NTRK3) was induced. Furthermore, it was confirmed that the expression of PPARγ and adiponectin, which are common markers for brown adipocytes and white adipocytes, was induced. On the other hand, it was also confirmed that the expression of the white adipocyte markers resistin, phosphoserine transaminase 1 (PSAT1), and endothelin receptor alpha (EDNRA) was not induced.

Resistin is a gene that not only elicits insulin resistance, but is also associated with canceration and arteriosclerosis. The fact that the expression of resistin is not induced in human pluripotent stem cell-derived brown adipocytes is an extremely important point in drug discovery studies using human pluripotent stem cell-derived brown adipocytes, and also when considering the safety of cell therapy using human pluripotent stem cell-derived brown adipocytes.

In addition, electron microscopic observations confirmed the presence of multilocular lipid droplets, which are microstructures characteristic of brown adipocytes, and vertically long fused mitochondria. Furthermore, ladder-shaped developed cristae characteristic of brown adipocytes were confirmed inside the mitochondria.

### [Industrial applicability]

The present invention has provided supernatants of brown adipocytes prepared from pluripotent stem cells and desalted purified products thereof. Further, the present invention has provided methods for preparing supernatants of brown adipocytes capable of improving metabolism without using a culture solution comprising amino acids, vitamins, a high concentration of glucose, and the like. The present invention has also provided methods for producing brown adipocytes derived from pluripotent stem cells, which are useful for preparing the supernatants of brown adipocytes. Through the methods of the present invention, it is possible to generate brown adipocytes without adding cytokines and obtain supernatants thereof.

The present invention enables stable provision of desalted and concentrated high-potency "supernatants of brown adipocytes" with a guaranteed "glucose metabolism-improving action", and no contamination with "synthetic cytokines", which were not obtainable by any means hitherto. The present invention also makes it possible to stably provide materials for searching BATokines in the fields of basic research and drug discovery, and provide high-potency BA-SUP dry products as BATokine cocktails maintaining various physiological activities (promotion of insulin secretion, promotion of glucose uptake) as a therapeutic tool for diseases of metabolic disorders such as obesity and type 2 diabetes.

Since the preparation of the high-potency BA-SUP dry products according to the present invention can be carried out in any country and region in the world, it can be developed into an industry with large-scale plants, is practical, and has a high industrial applicability.

## Claims

1. A method of producing a supernatant of brown adipocytes, or brown adipocytes that can be used for producing the supernatant of brown adipocytes, comprising the steps of:
(1) maintaining and culturing pluripotent stem cells feeder-free,
(2) dispersing the cells of step (1) and culturing them non-adhesively in a serum-free environment without adding any one of the cytokines of BMP4, BMP7, VEGF, SCF, FIt3L, IL6, and IGF2 to form cell aggregates, and
(3) culturing the cells of step (2) in a serum-free environment without adding any one of the cytokines of BMP7, VEGF, SCF, FIt3L, IL6, and IGF2.

2. The method according to claim 1, further comprising the step of:
(4) incubating the brown adipocytes of step (3) in a salt buffer and collecting the supernatant thereof.

3. The method according to claim 2, wherein the salt buffer is selected from Krebs-Ringer-Hepes (KRH), Krebs-Ringer-Tris (KRT), Krebs-Ringer Bicarbonate, Krebs-Ringer-Hepes-Bicarbonate (KRHB) or Krebs-Ringer-Tris Bicarbonate (KRTB).

4. The method according to claim 1, 2 or 3, wherein the salt buffer comprises glucose, preferably the salt buffer comprises about 2.8 mM glucose.

5. The method according to any one of claims 1 to 4, wherein in steps (2) and/or (3) the culturing is carried out without an exogenous cytokine.

6. The method according to any one of claims 1 to 5, wherein steps (2) and (3) are carried out with a medium of identical composition in the same culture vessel.

7. The method according to any one of claims 1 to 6, further comprising the step of desalting the obtained supernatant.

8. A composition comprising a supernatant of brown adipocytes produced by the method according to any one of claims 1 to 7.

9. The composition according to claim 8, wherein the composition does not comprise a cytokine cocktail comprising BMP7, VEGF, SCF, FIt3L, IL6, and IGF2.

10. The composition according to claim 8 or 9, wherein the composition does not comprise an exogenous cytokine.

11. The composition comprising the supernatant according to claim 8, wherein the composition is for a use in the treatment of a metabolic disease or a metabolic disorder, preferably wherein the metabolic disease or metabolic disorder is selected from the group consisting of obesity, overweightness, metabolic syndrome, and type 2 diabetes; a disease caused by a mitochondrial dysfunction, preferably wherein the mitochondrial dysfunction is selected from the group consisting of mitochondrial disease and neonatal heart disease; or a disease caused by skin damage, preferably wherein the skin damage is selected from the group consisting of subcutaneous bleeding, pigmentation due to subcutaneous bleeding, and subcutaneous telangiectasia.

## Patentansprüche

1. Verfahren zur Herstellung eines Überstands aus braunen Adipozyten oder braunen Adipozyten, die zur Herstellung des Überstands aus braunen Adipozyten verwendet werden kann, umfassend die Schritte:
(1) Erhaltung und Kultivierung pluripotenter Stammzellen ohne Feeder-Zellen,
(2) Dispergieren der Zellen des Schritts (1) und Kultivieren dieser nichtadhäsiv in einer serumfreien Umgebung ohne Zugabe eines der Zytokine von BMP4, BMP7, VEGF, SCF, FIt3L, IL6 und IGF2 zur Bildung von Zellaggregaten, und
(3) Kultivierung der Zellen von Schritt (2) in einer serumfreien Umgebung ohne Zugabe eines der Zytokine von BMP7, VEGF, SCF, FIt3L, IL6 und IGF2.

2. Verfahren nach Anspruch 1, das ferner den Schritt umfasst:
(4) Inkubieren der braunen Adipozyten der Stufe (3) in einem Salzpuffer und Sammeln des Überstands davon.

3. Verfahren nach Anspruch 2, wobei der Salzpuffer aus Krebs-Ringer-Hepes (KRH), Krebs-Ringer-Tris (KRT), Krebs-Ringer Bicarbonat, Krebs-Ringer-Hepes-Bicarbonat (KRHB) oder Krebs-Ringer-Tris Bicarbonat (KRTB) ausgewählt ist.

4. Verfahren nach Anspruch 1, 2 oder 3, wobei der Salzpuffer Glukose umfasst, vorzugsweise der Salzpuffer etwa 2,8 mM Glukose umfasst.

5. Verfahren nach einem der Ansprüche 1 bis 4, wobei in den Schritten (2) und/oder (3) die Kultivierung ohne ein exogenes Zytokin durchgeführt wird.

6. Verfahren nach einem der Ansprüche 1 bis 5, wobei die Schritte (2) und (3) mit einem Medium identischer Zusammensetzung in demselben Kulturgefäß durchgeführt werden.

7. Verfahren nach einem der Ansprüche 1 bis 6, das ferner den Schritt der Entsalzung des erhaltenen Überstands umfasst.

8. Eine Zusammensetzung umfassend einen Überstand brauner Adipozyten, hergestellt nach dem Verfahren nach einem der Ansprüche 1 bis 7.

9. Zusammensetzung nach Anspruch 8, wobei die Zusammensetzung keinen Zytokincocktail umfasst, der BMP7, VEGF, SCF, FIt3L, IL6 und IGF2 umfasst.

10. Zusammensetzung nach Anspruch 8 oder 9, wobei die Zusammensetzung kein exogenes Zytokin umfasst.

11. Zusammensetzung umfassend den Überstand nach Anspruch 8, wobei die Zusammensetzung für eine Verwendung zur Behandlung einer Stoffwechselerkrankung oder einer Stoffwechselstörung bestimmt ist, vorzugsweise wobei die Stoffwechselkrankheit oder Stoffwechselstörung aus der Gruppe ausgewählt wird, die aus Fettleibigkeit, Übergewicht, metabolischem Syndrom und Typ-2-Diabetes besteht; eine Krankheit, die durch eine mitochondriale Dysfunktion verursacht wird, vorzugsweise wobei die mitochondriale Dysfunktion aus der Gruppe ausgewählt wird, die aus der mitochondrialen Erkrankung und der neonatalen Herzkrankheit besteht; oder eine Krankheit, die durch Hautschäden verursacht wird, vorzugsweise wobei die Hautschädigung aus der Gruppe ausgewählt wird, die aus subkutanen Blutungen, Pigmentierung aufgrund von subkutanen Blutungen und subkutaner Teleangiektasie besteht.

## Revendications

1. Procédé de production d'un surnageant d'adipocytes bruns, ou d'adipocytes bruns pouvant être utilisé pour produire le surnageant d'adipocytes bruns, comprenant les étapes consistant à :
(1) maintenir et cultiver des cellules souches pluripotentes sans nourrisseur,
(2) disperser les cellules de l'étape (1) et les cultiver de manière non adhésive dans un environnement sans sérum sans ajouter l'une des cytokines de BMP4, BMP7, VEGF, SCF, FIt3L, IL6 et IGF2 pour former des agrégats cellulaires, et
(3) cultiver les cellules de l'étape (2) dans un environnement sans sérum sans ajouter l'une des cytokines de BMP7, VEGF, SCF, FIt3L, IL6 et IGF2.

2. Procédé selon la revendication 1, comprenant en outre l'étape consistant à :
(4) incubation des adipocytes bruns de l'étape (3) dans un tampon salin et collecte du surnageant de ceux-ci.

3. Procédé selon la revendication 2, dans lequel le tampon salin est choisi parmi Krebs-Ringer-Hepes (KRH), Krebs-Ringer-Tris (KRT), Krebs-Ringer Bicarbonate, Krebs-Ringer-Hepes-Bicarbonate (KRHB) ou Krebs-Ringer-Tris Bicarbonate (KRTB).

4. Procédé selon la revendication 1, 2 ou 3, dans lequel le tampon salin comprend du glucose, de préférence le tampon salin comprend environ 2,8 mM de glucose.

5. Procédé selon l'une quelconque des revendications 1 à 4, dans lequel, dans les étapes (2) et/ou (3), la culture est effectuée sans cytokine exogène.

6. Procédé selon l'une quelconque des revendications 1 à 5, dans lequel les étapes (2) et (3) sont réalisées avec un milieu de composition identique dans le même récipient de culture.

7. Procédé selon l'une quelconque des revendications 1 à 6, comprenant en outre l'étape de dessalage du surnageant obtenu.

8. Composition comprenant un surnageant d'adipocytes bruns produits par le procédé selon l'une quelconque des revendications 1 à 7.

9. Composition selon la revendication 8, dans laquelle la composition ne comprend pas un cocktail de cytokines comprenant BMP7, VEGF, SCF, FIt3L, IL6 et IGF2.

10. Composition selon la revendication 8 ou 9, dans laquelle la composition ne comprend pas de cytokine exogène.

11. Composition comprenant le surnageant selon la revendication 8, dans laquelle la composition est destinée à être utilisée dans le traitement d'une maladie métabolique ou d'un trouble métabolique, de préférence dans laquelle la maladie métabolique ou le trouble métabolique est choisi dans le groupe constitué par l'obésité, le surpoids, le syndrome métabolique et le diabète de type 2 ; une maladie causée par une dysfonction mitochondriale, de préférence dans laquelle la dysfonction mitochondriale est sélectionnée dans le groupe constitué de la maladie mitochondriale et de la cardiopathie néonatale ; ou une maladie causée par une lésion cutanée, de préférence dans laquelle les lésions cutanées sont sélectionnées dans le groupe comprenant les saignements sous-cutanés, la pigmentation due aux saignements sous-cutanés et les télangiectasies sous-cutanées.
